# EUROPEAN PATENT SPECIFICATION

(11) **EP 1 751 146 B1**
(45) Date of publication and mention of the grant of the patent: **02.12.2009**
(21) Application number: 05702134.7
(22) Date of filing: 07.02.2005
(51) Int. Cl.: C07D 417/00

(54) **PYRIDINYL- OR PYRIMIDINYL THIAZOLES WITH PROTEIN KINASE INHIBITING ACTIVITY**
PYRIDINYL- ODER PYRIMIDINYLTHIAZOLE MIT PROTEINKINASEHEMMENDER WIRKUNG
PYRIDINYL-OU PYRIMIDINYL THIAZOLES AYANT UNE ACTIVITE INHIBITRICE DES PROTEINES KINASES

(30) Priority: 06.02.2004 GB 0402653
(43) Date of publication of application: 14.02.2007
(73) Proprietor: Cyclacel Limited, London N1 7JQ (GB)
(72) Inventor: DUNCAN, Kenneth, Cambusbarron, Stirlingshire FK7 9RA (GB); GIBSON, Darren, Dundee DD5 3UN (GB); WANG, Shudong, Angus DD8 2RZ (GB); ZHELEVA, Daniella, Fife DD6 8NR (GB); FISCHER, Peter, Angus DD11 2EN (GB)
(74) Representative: Clyde-Watson, Zöe
(86) International application number: PCT/GB2005/000405
(87) International publication number: WO 2005/075468

(56) References cited:
- WO-A-01/72745
- WO-A-03/029248
- WO-A-2004/005283
- WO-A-2004/043953
- WO-A-2005/012298
- ZIMMERMANN J ET AL: "PHENYLAMINO-PYRIMIDINE (PAP) DERIVATIVES: A NEW CLASS OF POTENT ANDSELECTIVE INHIBITORS OF PROTEIN KINASE C (PKC)" July 1996 (1996-07), ARCHIV DER PHARMAZIE, VCH VERLAGSGESELLSCHAFT MBH, WEINHEIM, DE, PAGE(S) 371-376 , XP000885618 ISSN: 0365-6233 page 372 - page 373; tables I,II

## Description

The present invention relates to substituted pyrimidine derivatives. In particular, the invention relates to aryl-(4-thiazol-2-yl-pyrimidin-2-yl)-amines and aryl-(4-thiazol-2-yl-pyridin-2-yl)-amines and their use in therapy. More specifically, but not exclusively, the invention relates to compounds that are capable of inhibiting one or more protein kinases.

### BACKGROUND TO THE INVENTION

In eukaryotes, all biological functions, including DNA replication, cell cycle progression, energy metabolism, and cell growth and differentiation, are regulated through the reversible phosphorylation of proteins. The phosphorylation state of a protein determines not only its function, subcellular distribution, and stability, but also what other proteins or cellular components it associates with. The balance of specific phosphorylation in the proteome as a whole, as well as of individual members in a biochemical pathway, is thus used by organisms as a strategy to maintain homeostasis in response to an ever-changing environment. The enzymes that carry out these phosphorylation and dephosphorylation steps are protein kinases and phosphatases, respectively.

The eukaryotic protein kinase family is one of the largest in the human genome, comprising some 500 genes [1,2]. The majority of kinases contain a 250-300 amino acid residue catalytic domain with a conserved core structure. This domain comprises a binding pocket for ATP (less frequently GTP), whose terminal phosphate group the kinases transfers covalently to its macromolecular substrates. The phosphate donor is always bound as a complex with a divalent ion (usually Mg²⁺ or Mn²⁺). Another important function of the catalytic domain is the binding and orientation for phosphotransfer of the macromolecular substrate. The catalytic domains present in most kinases are more or less homologous.

A wide variety of molecules capable of inhibiting protein kinase function through antagonising ATP binding are known in the art [3-7]. By way of example, the applicant has previously disclosed 2-anilino-4-heteroaryl-pyrimidine compounds with kinase inhibitory properties, particularly against cyclin-dependent kinases (CDKs) [8-12]. CDKs are scrine/threonino protein kinases that associate with various cyclin subunits. These complexes are important for the regulation of eukaryotic cell cycle progression, but also for the regulation of transcription [13,14].

The present invention seeks to provide aryl-(4-thiazol-2-yl-pyrimidin-2-yl)-amines and aryl-(4-thiazol-2-yl-pyridin-2-yl)-amines. More specifically, the invention provides provides aryl-(4-thiazol-2-yl-pyrimidin 2-yl)-amines and aryl-(4-thiazol-2-yl-pyridin-2-yl)-amines which have broad therapeutic applications in the treatment of a number of different diseases and/or that are capable of inhibiting one or more protein kinases. ,

### STATEMENT OF INVENTION

A first aspect of the invention relates to a compound of formula **I**, or a pharmaceutically acceptable salt thereof 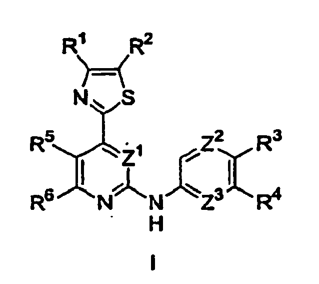 wherein:
Z¹ is N;
Z² is N;
Z³ is CR⁷;
R¹, R², R³, R⁴, R⁴, R⁶, and R⁷ are each independently H, R⁸, or R⁹;
each R⁸ is independently a C₁₋₆-alkyl group, a C₆₋₁₀-aryl group or a cycloheteroalkyl group, said cycloheteroalkyl group being a cyclic heteroalkyl group, wherein said heteroalkyl groups is a C₁₋₆-alkyl group comprising one or more heteroatoms;
each R⁹ is independently halo, NO₂, C₁₋₆-alkoxy, CN, CF₃, SO₃H, SO₂NR¹⁰R¹¹, SO₂R¹², NR¹³R¹⁴, (CH₂)ₐCOOR¹⁵, (CH₂)_{b}CONR¹⁶R¹⁷, (CH₂)_{c}COR¹⁸ or (CH₂)_{d}OH;
a, b, c and d are each independently 0, 1 2 3 or 4;
R¹⁰⁻¹⁸ are each independently H or C₁₋₆-alkyl.

A second aspect of the invention relates to compound of formula Ia, or a pharmaceutically acceptable salt thereof, 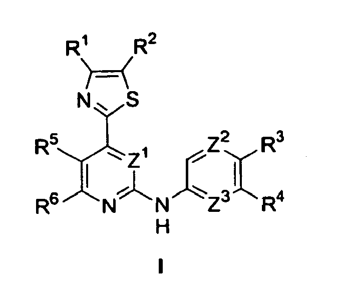 wherein:
Z¹ is N;
R¹ is alkyl, aryl, OH or (CH₂)ₐCOOR¹⁵;
R² is COR¹⁸, H, COOR¹⁵ or alkyl;
R³ is halo, H, OH, alkyl or morpholino;
R⁴ is H, NH₂, OH, CF₃ or NO₂; and
Z² and Z³ are both CH;
a is 0, 1 2 3 or 4;
R¹⁵ and R¹⁸ are each independently H or C₁₋₆-alkyl.

A third aspect of the invention relates to a pharmaceutical composition comprising a compound of formula I or Ia, or a pharmaceutically acceptable salt thereof, admixed with a pharmaceutically acceptable diluent, carrier or excipient.

A fourth aspect of the invention relates to a compound of formula I or Ia, or a pharmaceutically acceptable salt thereof, for use in medicine.

A fifth aspect of the invention relates to the use of a compound of formula I or Ia, or a pharmaceutically acceptable salt thereof, in the preparation of a medicament for treating one or more of the following disorders:
a proliferative disorder;
a viral disorder;
a CNS disorder;
diabetes;
stroke;
alopecia;
an inflammatory disease; or
an infectious disease.

A sixth aspect of the invention relates to the use of a compound of formula I or Ia, or a pharmaceutically acceptable salt thereof, in an assay for identifying candidate compounds capable of inhibiting one or more of a cyclin dependent kinase, aurora kinase, GSK and a PLK enzyme.

A seventh aspect of the invention relates to a process for preparing compounds of formulae I or Ia.

### DETAILED DESCRIPTION

As used herein, the term "hydrocarbyl" refers to a saturated or unsaturated, straight-chain, branched, or cyclic group comprising at least C and H that may optionally comprise one or more other suitable substituents. Examples of such substituents may include halo, CF₃, OH, CN, NO₂, SO₃H, SO₂NH₂, SO₂Me, NH₂, COOH, and CONH₂. If the hydrocarbyl group comprises more than one C then those carbons need not necessarily be linked to each other. For example, at least two of the carbons may be linked via a suitable element or group. Thus, the hydrocarbyl group may contain heteroatoms. Suitable heteroatoms will be apparent to those skilled in the art and include, for instance, sulphur, nitrogen, oxygen, phosphorus and silicon. Where the hydrocarbyl group contains one or more heteroatoms, the hydrocarbyl group may be connected to the rest of the molecule via a carbon-carbon bond or a carbon-heteroatom bond.

Preferably, the hydrocarbyl group is an aryl, alkyl, cycloheteroalkyl, cycloalkyl, heteroalkyl or heteroaryl group. More preferably still, the hydrocarbyl group is an aryl, alkyl or cycloheteroalkyl group.

As used herein the term "alkyl" includes both straight chain and branched alkyl groups. The alkyl group may be substituted (mono- or poly-) or unsubstituted. Suitable substituents include, for example, halo, CF₃, OH, CN, NO₂, SO₃H, SO₂NH₂, SO₂Me, NH₂, COOH, CONH₂ and alkoxy. Preferably, the alkyl group is a C₁₋₂₀ alkyl group, more preferably a C₁₋₁₅, more preferably still a C₁₋₁₂ alkyl group, more preferably still, a C₁₋₆ alkyl group, more preferably a C₁₋₃ alkyl group. Particularly preferred alkyl groups include, for example, methyl, ethyl, propyl, isopropyl, butyl, isobutyl, tert-butyl, pentyl and hexyl.

As used herein, the term "heteroalkyl" includes an alkyl group as defined above which comprises one or more heteroatoms.

As used herein, the term "cycloalkyl" refers to a cyclic alkyl group which may be substituted (mono- or poly-) or unsubstituted. Suitable substituents include, for example, halo, CF₃, OH, CN, NO₂, SO₃H, SO₂NH₂, SO₂Me, NH₂, COOH, CONH₂ and alkoxy.

Likewise, the term "cycloheteroalkyl" refers to a cyclic heteroalkyl group which may be substituted (mono- or poly-) or unsubstituted. Suitable substituents include, for example, halo, CF₃, OH, CN, NO₂, SO₃H, SO₂NH₂, SO₂Me, NH₂, COOH, CONH₂ and alkoxy. Preferred cycloheteroalkyl groups include morpholino, piperazinyl and piperidinyl groups.

As used herein, the term "aryl" refers to a C₆₋₁₀ aromatic, substituted (mono- or poly-) or unsubstituted group, and includes, for example, phenyl, naphthyl etc. Again, suitable substituents include, for example, halo, CF₃, OH, CN, NO₂, SO₃H, SO₂NH₂, SO₂Me, NH₂, COOH, CONH₂ and alkoxy.

As used herein, the term "heteroaryl" refers to a C₄₋₁₀ aromatic, substituted (mono- or poly-) or unsubstituted group, which comprises one or more heteroatoms. Preferred heteroaryl groups include pyrrole, pyrazole, pyrimidine, pyrazine, pyridine, quinoline, thiophene and furan. Again, suitable substituents include, for example, halo, CF₃, OH, CN, NO₂, SO₃H, SO₂NH₂, SO₂Me, NH₂, COOH, CONH₂ and alkoxy.

In one preferred embodiment of the invention, each R⁸ is independently a C₁₋₃₀ hydrocarbyl group, optionally containing up to twelve heteroatoms selected from N, S, and O, and optionally bearing up to six substituents each independently selected from halo, NO₂, CN, CF₃, SO₃H, SO₂NH₂ SO₂Me OH, NH₂, COOH, and CONH₂.

More preferably, each R⁸ is independently an alkyl group, an aryl group or a cycloheteroalkyl group. Preferably, the cycloheteroalkyl group is morpholinyl, pyrrolidinyl or piperidinyl.

Preferably, the cycloheteroalkyl group is N-morpholinyl, N-pyrrolidinyl or N-piperidinyl.

In one preferred embodiment of the invention, each R⁹ is independently halo, NO₂, alkoxy, CN, CF₃, SO₃H, SO₂NH₂, SO₂Me, OH, NH₂, (CH₂)ₐCOOR¹⁵, (CH₂)_{d}OH, CONH₂ or COR¹⁸.

In a more preferred embodiment of the invention, each R⁹ is independently halo, NO₂, alkoxy, CF₃, SO₃H, SO₂NH₂, SO₂Me, OH, NH₂, (CH₂)ₐCOOR¹⁵, (CH₂)_{d}OH, CONH₂ or COR¹⁸.

In a more preferred embodiment of the invention, each R⁹ is independently halo, NO₂, OMe, CF₃, SO₃H, SO₂NH₂, SO₂Me, OH, NH₂, CH₂COOMe, COOMe, COOEt, (CH₂)₂OH, CONH₂ or COMe.

In one preferred embodiment, R⁵ and R⁶ are both H and R¹⁻⁴ and R⁷ are each independently H, R⁸ or R⁹.

In another preferred embodiment, where Z² and Z³ are both CR⁷, at least one of R³, R⁴ and R⁷ is other than OMe.

In one particularly preferred embodiment,
R¹ is H, alkyl, aryl, (CH₂)ₐCOOR¹⁵ or OH;
R² is H, (CH₂)_{d}OH, (CH₂)ₐCOOR¹⁵, COR¹⁸ or alkyl;
R³ is halo, H, alkoxy, cycloheteroalkyl, alkyl or OH;
R⁴ is H, NH₂, OH, alkyl, CF₃ or NO₂; and
R⁵ and R⁶ are both H.
In one particularly preferred embodiment,
R¹ is H, Me, Ph, CH₂COOMe or OH;
R² is H, (CH₂)₂OH, COOEt, COMe or Me;
R³ is Cl, H, OMe, N-morpholinyl, N-pyrrolidinyl, Me or OH;
R⁴ is H, NH₂, OH, Me, CF₃ or NO₂; and
R⁵ and R⁶ are both H.

In another particularly preferred embodiment,
R¹ is H, alkyl, aryl, (CH₂)ₐCOOR¹⁵ or OH;
R² is H, COOR¹⁵, COR¹⁸ or alkyl;
**R³** is halo, H, alkoxy, morpholino, alkyl or OH;
R⁴ is H, NH₂, OH, CF₃ or NO₂; and
R⁵ and R⁶ are both H.

More preferably, for this embodiment,
R¹ is H, Me, Ph, CH₂CO₂Me or OH;
R² is H, CO₂Et, COMe or Me;
R³ is Cl, H, Some, morpholino, Me or OH.

More preferably still, R¹ is Me or OH, R² is COMe or Me, and R³ is OH or Cl.

More preferably still, R¹ is Me and R² is COMe.

In another preferred embodiment, Z¹ is N, Z² is N and Z³ is CR⁷. For this embodiment, more preferably,
R¹ is H, OH or alkyl;
R² is H, (CH₂)₄OH, alkyl, (CH₂)ₐCOOR¹⁵, COR¹⁸;
R³ is halo, alkoxy or heterocycloalkyl;
R⁴ is H or alkyl; and
Z³ is CH.

For this embodiment, more preferably,
R¹ is H, OH or Me;
R² is H, (CH₂)₂OH, Me, COOEt, COMe;
R³ is halo, OMe or N-pyrrolidinyl;
R⁴ is H or Me; and
Z³ is CH.

In another preferred embodiment,
R¹ is H or alkyl;
R² is H or COR¹⁸;
R³ is halo or alkoxy; and
Z³ is CH.

More preferably still,
R¹ is H or Me;
R² is H or COMe; and
R³ is halo or OMe.

In one especially preferred embodiment, the compound of formula I is selected from the following:
(6-Methoxy-pyridin-3-yl)-(4-thiazol-2-yl-pyrimidin-2-yl)-amine
(6-Chloro-pyridin-3-yl)-(4-thiazol-2-yl-pyrimidin-2-yl)-amine
1-{2-[2-(6-Chloro-pyridin-3-ylamino)-pyrimidin-4-yl]-4-methyl-thiazol-5-yl}-ethanone
[4-(4,5-Dimethyl-thiazol-2-yl)-pyrimidin-2-yl]-(6-methoxy-pyridin-3-yl)-amine
(6-Chloro-pyridin-3-yl)-[4-(4,5-dimethyl-thiazol-2-yl)-pyrimidin-2-yl]-amine
(6-Pyrrolidin-1-yl-pyridin-3-yl)-(4-thiazol-2-yl-pyrimidin-2-yl)-amine
2-[2-(6-Chloro-pyridin-3-ylamino)-pyrimidin-4-yl]-4-hydroxy-thiazole-5-carboxylic acid ethyl ester
2-[2-(6-Chloro-pyridin-3-ylamino)-pyrimidin-4-yl]-5-methyl-thiazol-4-ol
2-[2-(6-Chloro-pyridin-3-ylamino)-pyrimidin-4-yl]-5-(2-hydroxy-ethyl)-thiazol-4-ol
(6-Chloro-5-methyl-pyridin-3-yl)-(4-thiazol-2-yl-pyrimidin-2-yl)-amine.
N-[4-(4,5-Dimethyl-thiazol-2-yl)-pyrimidin-2-yl]-benzene-1,3-diamine
3-[4-(4,5-Dimethyl-thiazol-2-yl)-pyrimidin-2-ylamino]-phenol
[4-(4,5-Dimethyl-thiazol-2-yl)-pyrimidin-2-yl]-(3-trifluoromethyl-phenyl)-amine
(4-Chloro-3-trifluoromethyl-phenyl)-[4-(4,5-dimethyl-thiazol-2-yl)-pyrimidin-2-yl]-amine
[4-(4,5-Dimethyl-thiazol-2-yl)-pyrimidin-2-yl]-(3-nitro-phenyl)-amine
(6-Methoxy-pyridin-3-yl)-(4-thiazol-2-yl-pyrimidin-2-yl)-amine
(6-Chloro-pyridin-3-yl)-(4-thiazol-2-yl-pyrimidin-2-yl)-amine
1-{2-[2-(6-Chloro-pyridin-3-ylamino)-pyrimidin-4-yl]-4-methyl-thiazol-5-yl}-ethanone
[4-(4,5-Dimethyl-thiazol-2-yl)-pyrimidin-2-yl]-(6-methoxy-pyridin-3-yl)-amine
(6-Chloro-pyridin-3-yl)-[4-(4,5-dimethyl-thiazol-2-yl)-pyrimidin-2-yl]-amine
[4-(4,5-Dimethyl-thiazol-2-yl)-pyrimidin-2-yl]-(4-morpholin-4-yl-phenyl)-amine
[4-(4,5-Dimethyl-thiazol-2-yl)-pyrimidin-2-yl]-(4-methyl-3-nitro-phenyl)-amine
4-[4-(4,5-Dimethyl-thiazol-2-yl)-pyrimidin-2-ylamino]-phenol
2-[2-(4-Chloro-phenylamino)-pyridin-4-yl]-5-methyl-thiazol-4-ol
(6-Pyrrolidin-1-yl-pyridin-3-yl)-(4-thiazol-2-yl-pyrimidin-2-yl)-amine
2-[2-(6-Chloro-pyridin-3-ylamino)-pyrimidin-4-yl]-4-hydroxy-thiazole-5-carboxylic acid ethyl ester
2-[2-(6-Chloro-pyridin-3-ylamino)-pyrimidin-4-yl]-5-methyl-thiazol-4-ol
2-[2-(6-Chloro-pyridin-3-ylamino)-pyrimidin-4-yl]-5-(2-hydroxy-ethyl)-thiazol-4-ol
(6-Chloro-5-methyl-pyridin-3-yl)-(4-thiazol-2-yl-pyrimidin-2-yl)-amine.

In one particularly preferred embodment, the compound is selected from the following:
(6-Methoxy-pyridin-3-yl)-(4-thiazol-2-yl-pyrimidin-2-yl)-amine
(6-Chloro-pyridin-3-yl)-(4-thiazol-2-yl-pyrimidin-2-yl)-amine
[4-(4,5-Dimethyl-thiazol-2-yl)-pyrimidin-2-yl]-(6-methoxy-pyridin-3-yl)-amine
(6-Pyrrolidin-1-yl-pyridin-3-yl)-(4-thiazol-2-yl-pyrimidin-2-yl)-amine
2-[2-(6-Chloro-pyridin-3-ylamino)-pyrimidin-4-yl]-4-hydroxy-thiazole-5-carboxylic acid ethyl ester
2-[2-(6-Chloro-pyridin-3-ylamino)-pyrimidin-4-yl]-5-methyl-thiazol-4-ol
2-[2-(6-Chloro-pyridin-3-ylamino)-pyrimidin-4-yl]-5-(2-hydroxy-ethyl)-thiazol-4-ol
(6-Chloro-5-methyl-pyridin-3-yl)-(4-thiazol-2-yl-pyrimidin-2-yl)-amine.

More preferably, the compound of formula I is selected from the following:
(6-Methoxy-pyridin-3-yl)-(4-thiazol-2-yl-pyrimidin-2-yl)-amine; and
(6-Chloro-pyridin-3-yl)-(4-thiazol-2-yl-pyrimidin-2-yl)-amine
(6-Pyrrolidin-1-yl-pyridin-3-yl)-(4-thiazol-2-yl-pyrimidin-2-yl)-amine
2-[2-(6-Chloro-pyridin-3-ylamino)-pyrimidin-4-yl]-4-hydroxy-thiazole-5-carboxylic acid ethyl ester
2-[2-(6-Chloro-pyridin-3-ylamino)-pyrimidin-4-yl]-5-methyl-thiazol-4-ol
2-[2-(6-Chloro-pyridin-3-ylamino)-pyrimidin-4-yl]-5-(2-hydroxy-ethyl)-thiazol-4-ol
(6-Chloro-5-methyl-pyridin-3-yl)-(4-thiazol-2-yl-pyrimidin-2-yl)-amine

In one highly preferred embodiment of the invention, the compound of formula I is (6-Chloro-pyridin-3-yl)-(4-thiazol-2-yl-pyrimidin-2-yl)-amine.

Another embodiment of the invention relates to a compound of formula Ia, or a pharmaceutically acceptable salt thereof, 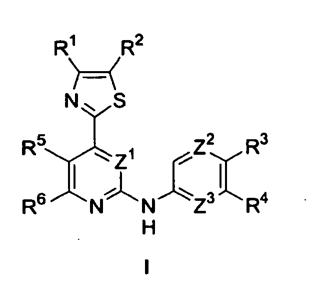 wherein:
Z¹ is N;
R¹ is alkyl, aryl, OH or (CH₂)ₐCOOR¹⁵;
R² is COR¹⁸, H, COOR¹⁵ or alkyl;
R³ is halo, H, OH, alkyl or morpholino;
R⁴ is H, NH₂, OH, CF₃ or NO₂; and
Z² and Z³ are both CH;
a is 0,1 2 3 or 4;
R¹⁵ and R¹⁸ are each independently H or C₁₋₆-alkyl.

Preferably,
R¹ is Me, Ph, OH or CH₂COOMe;
R² is COMe, H, COOEt or Me; and
R³ is halo, H, OH, C₁₋₆-alkyl or morpholino.

Even more preferably, the compound is selected from
1-{2-[2-(4-Chloro-phenylamino)-pyrimidin-4-yl]-4-methyl-thiazol-5-yl} -ethanone
(4-Chloro-phenyl)-[4-(4-methyl-thiazol-2-yl)-pyrimidin-2-yl]-amine
(4-Chloro-phenyl)-[4-(4-phenyl-thiazol-2-yl)-pyrimidin-2-yl]-amine
2-[2-(4-Chloro-phenylamino)-pyrimidin-4-yl]-4-methyl-thiazole-5-carboxylic acid ethyl ester
2-[2-(4-Chloro-phenylamino)-pyrimidin-4-yl]-4-methyl-thiazole-5-carboxylic acid ethyl ester
{2-[2-(4-Chloro-phenylamino)-pyrimidin-4-yl]-thiazol-4-yl}-acetic acid methyl ester
2-[2-(4-Chloro-phenylamino)-pyrimidin-4-yl]-4-hydroxy-thiazole-5-carboxylic acid ethyl ester
N-[4-(4,5-Dimethyl-thiazol-2-yl)-pyrimidin-2-yl]-benzene-1,3-diamine
3-[4-(4,5-Dimethyl-thiazol-2-yl)-pyrimidin-2-ylamino]-phenol
[4-(4,5-Dimethyl-thiazol-2-yl)-pyrimidin-2-yl]-(3-trifluoromethyl-phenyl)-amine
(4-Chloro-3-trifluoromethyl-phenyl)-[4-(4,5-dimethyl-thiazol-2-yl)-pyrimidin-2-yl]-amine
[4-(4,5-Dimethyl-thiazol-2-yl)-pyrimidin-2-yl]-(3-nitro-phenyl)-amine
[4-(4,5-Dimethyl-thiazol-2-yl)-pyrimidin-2-yl]-(4-morpholin-4-yl-phenyl)-amine
[4-(4,5-Dimethyl-thiazol-2-yl)-pyrimidin-2-yl]-(4-methyl-3-nitro-phenyl)-amine
4-[4-(4,5-Dimethyl-thiazol-2-yl)-pyrimidin-2-ylamino]-phenol.

In one preferred embodiment, the compound of the invention is capable of inhibiting one or more kinases selected from those set forth in Tables 5 or 6.

In one particularly preferred embodiment, the compound of the invention is capable of inhibiting one or more kinases selected from a cyclin dependent kinase or GSK. More preferably, the compound is capable of inhibiting one or more of GSK3, CDK2/E, CDK2/A, CDK1/B, CDK4/D1, CDK7/H and/or CDK9/T1.

Preferably, the compound of the invention has an IC₅₀ value for inhibition of one of the above-mentioned kinases of less than 10 µM, more preferably, less than 5 µM, more preferably still, less than 1 µM, even more preferably less than 0.1 µM, more preferably still less than 0.01 µM, as measured by the appropriate kinase assay. Details of suitable assays are outlined in the accompanying examples section.

In one particularly preferred embodiment, the compound of the invention is capable of selectively inhibiting GSK (preferably GSK3) over one or more cyclin dependent kinases selected from CDK2/E, CDK2/A, CDK1/B, CDK4/D1, CDK7/H and CDK9/T1. Preferably, the compound exhibits at least a 5-fold selectivity for GSK over CDK, more preferably at least a 10-fold selectivity, more preferably still at least a 100-fold selectivity for GSK. In one especially preferred embodiment, the compound exhibits at least a 1000-fold selectivity for GSK over CDK, more preferably at least 5000-fold selectivity.

In one particularly preferred embodiment, the compound exhibits at least a 10-fold selectivity for GSK3 over a CDK selected from CDK2/cyclin E, CDK1/cyclin B, CDK7/cyclin H, CDK4/cyclin D1, CDK2/cyclin A and CDK9/cyclin T1.

In another particularly preferred embodiment, the compound exhibits at least a 100-fold selectivity for GSK3 over a CDK selected from CDK2/cyclin E, CDK1/cyclin B, CDK7/cyclin H, CDK4/cyclin D1 and CDK2/cyclin A.

In yet another particularly preferred embodiment, the compound exhibits at least a 1000-fold selectivity for GSK3 over a CDK selected from CDK1/cyclin B, CDK7/cyclin H, CDK4/cyclin D1 and CDK2/cyclin A.

In another preferred embodiment, the compound of the invention is capable of activating cellular glycogen synthase activity. Preferably, the compound is capable of activating cellular glycogen synthase activity as measured by monitoring the fold induction of GS activity in HEK293, mouse myocyte or mouse adipocyte cells. Preferably, GS activity is activated by at least 1.5-fold, more preferably at least 2-fold, more preferably still at least 3-fold, 4-fold or 5-fold.

### PHARMACEUTICAL COMPOSITIONS

In one preferred embodiment of the invention, the compound of formula I is administered in combination with a pharmaceutically acceptable excipient, diluent or carrier.

Even though the compounds of the present invention (including their pharmaceutically acceptable salts, esters and pharmaceutically acceptable solvates) can be administered alone, they will generally be administered in admixture with a pharmaceutical carrier, excipient or diluent, particularly for human therapy. The pharmaceutical compositions may be for human or animal usage in human and veterinary medicine.

Examples of such suitable excipients for the various different forms of pharmaceutical compositions described herein may be found in the "handbook of Pharmaceutical Excipients, 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s).

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

### SALTS/ESTERS

The compounds of formula I can be present as salts or esters, in particular pharmaceutically acceptable salts or esters.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

### ENANTIOMERS/TAUTOMERS

In all aspects of the present invention previously discussed, the invention includes, where appropriate all enantiomers and tautomers of compounds of formula I. The man skilled in the art will recognise compounds that possess an optical properties (one or more chiral carbon atoms) or tautomeric characteristics. The corresponding enantiomers and/or tautomers may be isolated/prepared by methods known in the art.

### STEREO AND GEOMETRIC ISOMERS

Some of the specific compounds of formula I may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the compound or a pharmaceutically acceptable salt thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as 2H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. Isotopic variations of the agent of the present invention and pharmaceutically acceptable salts thereof of this invention can generally be prepared by conventional procedures using appropriate isotopic variations of suitable reagents.

### SOLVATES

The present invention also includes the use of solvate forms of the compounds of the present invention. The terms used in the claims encompass these forms.

### POLYMORPHS

The invention furthermore relates to the compounds of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### PRODRUGS

The invention further includes the compounds of the present invention in prodrug form. Such prodrugs are generally compounds of formula I wherein one or more appropriate groups have been modified such that the modification may be reversed upon administration to a human or mammalian subj ect. Such reversion is usually performed by an enzyme naturally present in such subject, though it is possible for a second agent to be administered together with such a prodrug in order to perform the reversion in vivo. Examples of such modifications include ester (for example, any of those described above), wherein the reversion may be carried out be an esterase etc. Other such systems will be well known to those skilled in the art.

### THERAPEUTIC USE

The compounds of formula I have been found to possess anti-proliferative activity and are therefore believed to be of use in the treatment of proliferative disorders such as cancers, leukaemias and other disorders associated with uncontrolled cellular proliferation such as psoriasis and restenosis. As defined herein, an anti-proliferative effect within the scope of the present invention may be demonstrated by the ability to inhibit cell proliferation in an *in vitro* whole cell assay, for example using any of the cell lines A549, HT29 or Saos-2 Using such assays it may be determined whether a compound is anti-proliferative in the context of the present invention.

On preferred embodiment of the present invention therefore relates to the use of one or more compounds of formula I in the preparation of a medicament for treating a proliferative disorder.

As used herein the phrase "preparation of a medicament" includes the use of a compound of formula I directly as the medicament in addition to its use in a screening programme for further therapeutic agents or in any stage of the manufacture of such a medicament

Preferably, the proliferative disorder is a cancer or leukaemia. The term proliferative disorder is used herein in a broad sense to include any disorder that requires control of the cell cycle, for example cardiovascular disorders such as restenosis, cardiomyopathy and myocardial infarction, auto-immune disorders such as glomerulonephritis and rheumatoid arthritis, dermatological disorders such as psoriasis, anti-inflammatory, anti-fungal, antiparasitic disorders such as malaria, emphysema, alopecia, and chronic obstructive pulmonary disorder. In these disorders, the compounds of the present invention may induce apoptosis or maintain stasis within the desired cells as required.

The compounds of the invention may inhibit any of the steps or stages in the cell cycle, for example, formation of the nuclear envelope, exit from the quiescent phase of the cell cycle (G0), G1 progression, chromosome decondensation, nuclear envelope breakdown, START, initiation of DNA replication, progression of DNA replication, termination of DNA replication, centrosome duplication, G2 progression, activation of mitotic or meiotic functions, chromosome condensation, centrosome separation, microtubule nucleation, spindle formation and function, interactions with microtubule motor proteins, chromatid separation and segregation, inactivation of mitotic functions, formation of contractile ring, and cytokinesis functions. In particular, the compounds of the invention may influence certain gene functions such as chromatin binding, formation of replication complexes, replication licensing, phosphorylation or other secondary modification activity, proteolytic degradation, microtubule binding, actin binding, septin binding, microtubule organising centre nucleation activity and binding to components of cell cycle signalling pathways.

In one embodiment of the invention, the compound of formula I is administered in an amount sufficient to inhibit at least one CDK enzyme.

Preferably, the compound of formula I is administered in an amount sufficient to inhibit at least one of CDK2 and/or CDK4.

Another aspect of the invention relates to the use of a compound of formula I in the preparation of a medicament for treating a viral disorder, such as human cytomegalovirus (HCMV), herpes simplex virus type 1 (HSV-1), human immunodeficiency virus type 1 (HIV-1), and varicella zoster virus (VZV).

In a more preferred embodiment of the invention, the compound of formula I is administered in an amount sufficient to inhibit one or more of the host cell CDKs involved in viral replication, *i.e*. CDK2, CDK7, CDK8, and CDK9 [39].

As defined herein, an anti-viral effect within the scope of the present invention may be demonstrated by the ability to inhibit CDK2, CDK7, CDK8 or CDK9.

In a particularly preferred embodiment, the invention relates to the use of one or more compounds of formula I in the treatment of a viral disorder which is CDK dependent or sensitive. CDK dependent disorders are associated with an above normal level of activity of one or more CDK enzymes. Such disorders preferably associated with an abnormal level of activity of CDK2, CDK7, CDK8 and/or CDK9. A CDK sensitive disorder is a disorder in which an aberration in the CDK level is not the primary cause, but is downstream of the primary metabolic aberration. In such scenarios, CDK2, CDK7, CDK8 and/or CDK9 can be said to be part of the sensitive metabolic pathway and CDK inhibitors may therefore be active in treating such disorders.

Another aspect of the invention relates to the use of compounds of formula I, or pharmaceutically accetable salts thereof, in the preparation of a medicament for treating diabetes.

In a particularly preferred embodiment, the diabetes is type II diabetes.

Glycogen synthase kinase 3 (GSK3) is a Ser/Thr protein kinase composed of two isoforms (α and β), which are highly homologous within the catalytic domain. GSK3 is one of several protein kinases that phosphorylate glycogen synthase (GS). The stimulation of glycogen synthesis by insulin in skeletal muscle results from the dephosphorylation and activation of GS. The action of GSK3 on GS thus results in the deactivation of the latter, thereby suppressing the conversion of glucose into glycogen in muscles.

Type II diabetes (non-insulin dependent diabetes mellitus) is a multi-factorial disease. Hyperglycaemia is due to insulin resistance in the liver, muscles, and other tissues, coupled with impaired secretion of insulin. Skeletal muscle is the main site for insulin-stimulated glucose uptake, there it is either removed from circulation or converted to glycogen. Muscle glycogen deposition is the main determinant in glucose homeostasis and type II diabetics have defective muscle glycogen storage. There is evidence that an increase in GSK3 activity is important in type II diabetes [1]. Furthermore, it has been demonstrated that GSK3 is over-expressed in muscle cells of type II diabetics and that an inverse correlation exists between skeletal muscle GSK3 activity and insulin action [2].

GSK3 inhibition may therefore be of therapeutic relevance in the treatment of diabetes, particularly type II, and diabetic neuropathy. For a recent review on GSK3 biology refer to [3]. It should be noted that GSK3 is known to phosphorylate many substrates other than GS and is thus involved in the regulation of multiple biochemical pathways.

GSK-3 substrates include: CREB (also known as cAMP response element-binding protein 1), which is involved in mediating gene transcription subsequent to increased cAMP levels and cAMP-dependent protein kinase A activation. The response element to which CREB binds is found in a number of genes, including those that are of proposed importance to T cell function (and dysfunction - for example T cell lymphoma and leukemia). CREB also appears to be involved in long-term potentiation in hippocampal CA1 neurons and in the regulation of neural function in general, as well as in cancer biology. EIF2B (eukaryotic initiation factor-2B), which is a GTP exchange protein, essential for protein synthesis. HSF-1 (heat-shock factor-1), which is a component of the cellular response to stress. C/EBPa (also known as CCAAT/enhancer-binding protein a), which has been suggested to modulate leptin expression and has also been suggested to have a function in human obesity. Mice homozygous for the targeted deletion of the C/E7Pa gene do not store hepatic glycogen, express low levels of GS and fail to store lipid. NF-ATc (nuclear factor of activated T cells), whose activation is controlled by calcineurin, a Ca²⁺-dependent phosphatase. Originally identified in T cells as inducers of cytokine gene expression, NF-AT proteins play varied roles in non-immune processes, particularly those related to adaptive responses such as cardiac hypertrophy and altered metabolic balance. c-Jun, c-myc and c-myb, each of which are protooncogenes. β-Catenin, which is a protein found in the adherens junction and is therefore critical for the establishment and maintenance of epithelial layers. Junctions mediate adhesion between cells, facilitate cell-cell signalling, and anchor the actin cytoskeleton. In serving these roles, adherens junctions regulate normal cell growth and behaviour, wound healing, and tumour cell metastasis. Tau, which is perhaps best known for its proposed involvement in the etiology of Alzheimer's disease. Tau co-assembles with tubulin into microtubules, however in Alzheimer's disease, tau forms large tangles of filaments, which disrupt the microtubule structures in the nerve cell, impairing the transport of nutrients as well as the transmission of neuronal messages. Insulin receptor substrate-1 (IRS-1), which is found in a variety of insulin responsive cells and tissues. It exhibits no intrinsic enzyme activity but is believed to serve as a docking protein involved in binding and activating other signal transduction molecules after being phosphorylated by the insulin receptor kinase. IRS-1 has been proposed to play a role in the development of insulin resistance.

It is notable that GSK3 is known to phosphorylate many substrates other than GS, and is thus involved in the regulation of multiple biochemical pathways. For example, GSK is highly expressed in the central and peripheral nervous systems and biomedical rationales for therapy through GSK inhibition in neurodegenerative diseases have been proposed.

Another aspect of the invention therefore relates to the use of compounds of formula I, or pharmaceutically acceptable salts thereof, in the preparation of a medicament for treating a CNS disorders, for example neurodegenerative disorders.

Preferably, the CNS disorder is Alzheimer's disease.

Tau is a GSK-3 substrate which has been, implicated in the etiology of Alzheimer's disease. In healthy nerve cells, Tau co-assembles with tubulin into microtubules. However, in Alzheimer's disease, tau forms large tangles of filaments, which disrupt the microtubule structures in the nerve cell, thereby impairing the transport of nutrients as well as the transmission of neuronal messages.

Without wishing to be bound by theory, it is believed that GSK3 inhibitors may be able to prevent and/or reverse the abnormal hyperphosphorylation of the microtubule-associated protein tau that is an invariant feature of Alzheimer's disease and a number of other neurodegenerative diseases, such as progressive supranuclear palsy, corticobasal degeneration and Pick's disease. Mutations in the tau gene cause inherited forms of fronto-temporal dementia, further underscoring the relevance of tau protein dysfunction for the neurodegenerative process [40].

Another aspect of the invention relates to the use of compounds of formula I, or pharmaceutically acceptable salts thereof, in the preparation of a medicament for treating bipolar disorder.

Yet another aspect of the invention relates to the use of compounds of formula I, or pharmaceutically acceptable salts thereof, in the preparation of a medicament for treating a stroke.

Reducing neuronal apoptosis is an important therapeutic goal in the context of head trauma, stroke, epilepsy, and motor neuron disease [4]. Therefore GSK3 as a pro-apoptotic factor in neuronal cells makes this protein kinase an attractive therapeutic target for the design of inhibitory drugs to treat these diseases. GSK3 inhibitors may be able to prevent and/or reverse the abnormal hyperphosphorylation of the microtubule-associated protein tau that is an invariant feature of Alzheimer's disease and a number of other neurodegenerative diseases, such as progressive supranuclear palsy, corticobasal degeneration and Pick's disease. Mutations in the tau gene cause inherited forms of fronto-temporal dementia, further underscoring the relevance of tau protein dysfunction for the neurodegenerative process [5].

Yet another aspect of the invention relates to the use of compounds of formula I, or pharmaceutically acceptable salts thereof, in the preparation of a medicament for treating alopecia.

Hair growth is controlled by the Wnt signalling pathway, in particular Wnt-3. In tissue-culture model systems of the skin, the expression of non-degradable mutants of β-catenin leads to a dramatic increase in the population of putative stem cells, which have greater proliferative potential [6]. This population of stem cells expresses a higher level of non-cadherin-associated β-catenin [7], which may contribute to their high proliferative potential. Moreover, transgenic mice overexpressing a truncated β-catenin in the skin undergo *de novo* hair-follicle morphogenesis, which normally is only established during embryogenesis. This raises the possibility that ectopic application of GSK3 inhibitors might be of use in the treatment of baldness and in restoring hair growth following chemotherapy-induced alopecia.

A further aspect of the invention relates to a method of treating a GSK3-dependent disorder, said method comprising administering to a subject in need thereof, a compound of formula I, or a pharmaceutically acceptable salt thereof, as defined above in an amount sufficient to inhibit GSK3.

Preferably, the compound of formula I, or pharmaceutically acceptable salt thereof, is administered in an amount sufficient to inhibit GSK3β.

In one embodiment of the invention, the compound of formula I is administered in an amount sufficient to inhibit at least one PLK enzyme.

The polo-like kinases (PLKs) constitute a family of serine/threonine protein kinases. Mitotic *Drosophila melanogaster* mutants at the *polo* locus display spindle abnormalities [41] and *polo* was found to encode a mitotic kinase [42]. In humans, there exist three closely related PLKs [43]. They contain a highly homologous amino-terminal catalytic kinase domain and their carboxyl termini contain two or three conserved regions, the polo boxes. The function of the polo boxes remains incompletely understood but they are implicated in the targeting of PLKs to subcellular compartments [44,45], mediation of interactions with other proteins [46], or may constitute part of an autoregulatory domain [47]. Furthermore, the polo box-dependent PLK1 activity is required for proper metaphase/anaphase transition and cytokinesis [48,49].

Studies have shown that human PLKs regulate some fundamental aspects of mitosis [50,51]. In particular, PLK1 activity is believed to be necessary for the functional maturation of centrosomes in late G2/early prophase and subsequent establishment of a bipolar spindle. Depletion of cellular PLK1 through the small interfering RNA (siRNA) technique has also confirmed that this protein is required for multiple mitotic processes and completion of cytokinesis [52].

In a more preferred embodiment of the invention, the compound of formula I is administered in an amount sufficient to inhibit PLK1.

Of the three human PLKs, PLK1 is the best characterized; it regulates a number of cell division cycle effects, including the onset of mitosis [53,54], DNA-damage checkpoint activation [55,56], regulation of the anaphase promoting complex [57-59], phosphorylation of the proteasome [60], and centrosome duplication and maturation [61].

Specifically, initiation of mitosis requires activation of M-phase promoting factor (MPF), the complex between the cyclin dependent kinase CDK1 and B-type cyclins [62]. The latter accumulate during the S and G2 phases of the cell cycle and promote the inhibitory phosphorylation of the MPF complex by WEE1, MIK1, and MYT1 kinases. At the end of the G2 phase, corresponding dephosphorylation by the dual-specificity phosphatase CDC25C triggers the activation of MPF [63]. In interphase, cyclin B localizes to the cytoplasm [64], it then becomes phosphorylated during prophase and this event causes nuclear translocation [65,66]. The nuclear accumulation of active MPF during prophase is thought to be important for initiating M-phase events [67]. However, nuclear MPF is kept inactive by WEE1 unless counteracted by CDC25C. The phosphatase CDC25C itself, localized to the cytoplasm during interphase, accumulates in the nucleus in prophase [68-71]. The nuclear entry of both cyclin B [60] and CDC25C [72] are promoted through phosphorylation by PLK1 [54]. This kinase is an important regulator of M-phase initiation.

In one particularly preferred embodiment, the compounds of formula I are ATP-antagonistic inhibitors of PLK1.

In the present context ATP antagonism refers to the ability of an inhibitor compound to diminish or prevent PLK catalytic activity, *i.e.* phosphotransfer from ATP to a macromolecular PLK substrate, by virtue of reversibly or irreversibly binding at the enzyme's active site in such a manner as to impair or abolish ATP binding.

In another preferred embodiment, the compound of formula I is administered in an amount sufficient to inhibit PLK2 and/or PLK3.

Mammalian PLK2 (also known as SNK) and PLK3 (also known as PRK and FNK) were originally shown to be immediate early gene products. PLK3 kinase activity appears to peak during late S and G2 phase. It is also activated during DNA damage checkpoint activation and severe oxidative stress. PLK3 also plays an important role in the regulation of microtubule dynamics and centrosome function in the cell and deregulated PLK3 expression results in cell cycle arrest, and apoptosis [73]. PLK2 is the least well understood homologue of the three PLKs. Both PLK2 and PLK3 may have additional important post-mitotic functions [46].

### ADMINISTRATION

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules. Preferably, these compositions contain from 1 to 250 mg and more preferably from 10-100 mg, of active ingredient per dose.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention, may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10 - 1000 mg, preferably between 10 - 250 mg, of active ingredient per dose.

Compositions may be formulated in unit dosage form, i.e., in the form of discrete portions containing a unit dose, or a multiple or sub-unit of a unit dose.

### DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

Depending upon the need, the agent may be administered at a dose of from 0.01 to 30 mg/kg body weight, such as from 0.1 to 10 mg/kg, more preferably from 0.1 to 1 mg/kg body weight.

In an exemplary embodiment, one or more doses of 10 to 150 mg/day will be administered to the patient for the treatment of malignancy.

### COMBINATIONS

In a particularly preferred embodiment, the one or more compounds of formula I are administered in combination with one or more other active agents, for example, existing drugs available on the market In such cases, the compounds of the invention may be administered consecutively, simultaneously or sequentially with the one or more other active agents.

Anticancer drugs in general are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance in early tumor cells which would have been otherwise responsive to initial chemotherapy with a single agent. An example of the use of biochemical interactions in selecting drug combinations is demonstrated by the administration of leucovorin to increase the binding of an active intracellular metabolite of 5-fluorouracil to its target, thymidylate synthase, thus increasing its cytotoxic effects.

Numerous combinations are used in current treatments of cancer and leukemia. A more extensive review of medical practices may be found in "Oncologic Therapies" edited by E. E. Vokes and H. M. Golomb, published by Springer.

Beneficial combinations may be suggested by studying the growth inhibitory activity of the test compounds with agents known or suspected of being valuable in the treatment of a particular cancer initially or cell lines derived from that cancer. This procedure can also be used to determine the order of administration of the agents, i.e. before, simultaneously, or after delivery. Such scheduling may be a feature of all the cycle acting agents identified herein.

### ASSAYS

Another aspect of the invention relates to the use of a compound of formula I, or a pharmaceutically acceptable salt thereof, as defined hereinabove in an assay for identifying further candidate compounds that influence the activity of a kinase selected from a cyclin dependent kinase, aurora kinase, GSK and a polo-like kinase.

Preferably, the assay is capable of identifying candidate compounds that are capable of inhibiting a kinase selected from a cyclin dependent kinase, aurora kinase, GSK and a polo-like kinase.

More preferably, the assay is a competitive binding assay.

As used herein, the term "candidate compound" includes, but is not limited to, a compound which may be obtainable from or produced by any suitable source, whether natural or not.

The candidate compound may be designed or obtained from a library of compounds, which may comprise peptides, as well as other compounds, such as small organic molecules and particularly new lead compounds. By way of example, the candidate compound may be a natural substance, a biological macromolecule, or an extract made from biological materials - such as bacteria, fungi, or animal (particularly mammalian) cells or tissues, an organic or an inorganic molecule, a synthetic candidate compound, a semi-synthetic candidate compound, a structural or functional mimetic, a peptide, a peptidomimetic, a derivatised candidate compound, a peptide cleaved from a whole protein, or a peptide synthesised synthetically, for example, either using a peptide synthesiser or by recombinant techniques or combinations thereof, a recombinant candidate compound, a natural or a non-natural candidate compound, a fusion protein or equivalent thereof and mutants, derivatives or combinations thereof. The candidate compound may even be a compound that is a modulator of a cyclin dependent kinase, aurora kinase, GSK or a polo-like kinase, such as a known inhibitor that has been modified in some way eg. by recombinant DNA techniques or chemical synthesis techniques.

Typically, the candidate compound will be prepared by recombinant DNA techniques and/or chemical synthesis techniques.

Once a candidate compound capable of interacting with a cyclin dependent kinase, aurora kinase, GSK or a polo-like kinase, has been identified, further steps may be carried out to select and/or to modify the candidate compounds and/or to modify existing compounds, such that they are able to modulate a cyclin dependent kinase, aurora kinase, GSK or a polo-like kinase,

Preferably, the candidate compound is generated by conventional SAR modification of a compound of the invention.

As used herein, the term "conventional SAR modification" refers to standard methods known in the art for varying a given compound by way of chemical derivatisation.

Thus, in one aspect, the identified compound may act as a model (for example, a template) for the development of other compounds. The compounds employed in such a test may be free in solution, affixed to a solid support, borne on a cell surface, or located intracellularly. The abolition of activity or the formation of binding complexes between the compound and the agent being tested may be measured.

The assay of the present invention may be a screen, whereby a number of agents are tested. In one aspect, the assay method of the present invention is a high through-put screen.

This invention also contemplates the use of competitive drug screening assays in which neutralising antibodies capable of binding a compound specifically compete with a candidate compound for binding to a compound.

Another technique for screening provides for high throughput screening (HTS) of agents having suitable binding affinity to the substances and is based upon the method described in detail in WO 84/03564.

It is expected that the assay methods of the present invention will be suitable for both small and.large-scale screening of test compounds as well as in quantitative assays.

One aspect of the invention relates to a process comprising the steps of:
(a) performing an assay method described hereinabove;
(b) identifying one or more candidate compounds capable of binding to a cyclin dependent kinase, aurora kinase, GSK or a polo-like kinase; and
(c) preparing a quantity of said one or more candidate compounds.

Another aspect of the invention provides a process comprising the steps of:
(a) performing an assay method described hereinabove;
(b) identifying one or more candidate compounds capable of binding to a cyclin dependent kinase, aurora kinase, GSK or a polo-like kinase; and
(c) preparing a pharmaceutical composition comprising said one or more candidate compounds.

Another aspect of the invention provides a process comprising the steps of:
(a) performing an assay method described hereinabove;
(b) identifying one or more candidate compounds capable of binding to a cyclin dependent kinase, aurora kinase, GSK or a polo-like kinase;
(c) modifying said one or more candidate compounds capable of binding to a cyclin dependent kinase, aurora kinase, GSK or a polo-like kinase;
(d) performing the assay method described hereinabove;
(e) optionally preparing a pharmaceutical composition comprising said one or more candidate compounds.

The invention also relates to candidate compounds identified by the method described hereinabove.

Yet another aspect of the invention relates to a pharmaceutical composition comprising a candidate compound identified by the method described hereinabove.

Another aspect of the invention relates to the use of a candidate compound identified by the method described hereinabove in the preparation of a pharmaceutical composition for use in the treatment of proliferative disorders.

The above methods may be used to screen for a candidate compound useful as an inhibitor of a cyclin dependent kinase, aurora kinase, GSK or a polo-like kinase.

### SYNTHESIS

Another aspect of the invention relates to a process for preparing compounds of formula I, said process comprising reacting a compound of formula 9 with a compound of formula 10 to form a compound of formula I, wherein R¹⁻⁶ are as defined above.

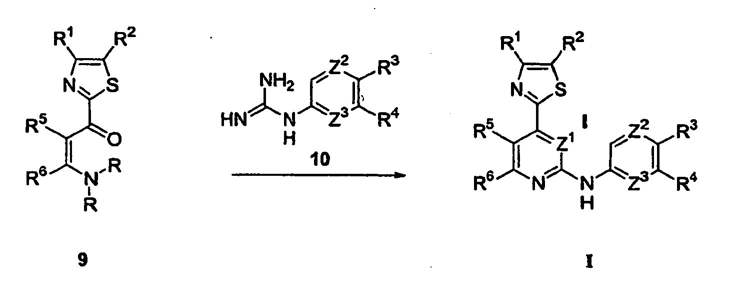

Yet another aspect of the invention relates to an alternative process for preparing compounds of formula I, said process comprising reacting a compound of formula 15 with a compound of formula 3 to form a compound of formula I, wherein R¹⁻⁶ are as defined above.

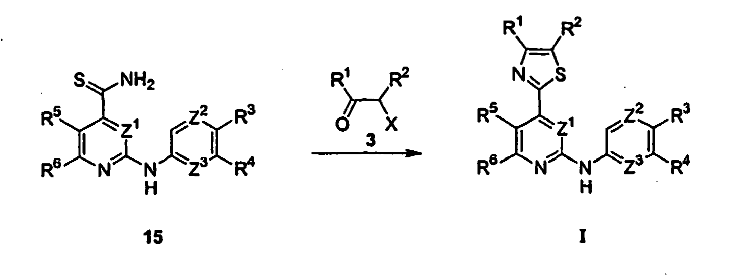

Compounds of general structure **I** in which Z¹ is N, *i.e.* 2,4-disubstituted pyrimidines, can be prepared by variations of the Traube pyrimidine synthesis [8,9] (*Scheme 1*). In these procedures the pyrimidine ring in **I** (Z¹ = N) is formed by condensation of 1,3-dicarbonyl compounds **8** or the corresponding enaminones **9** (where R is *e.g*. Me) with arylguanidines **10** [10]. The latter can be prepared from arylamines **11** by a variety of methods [11,12], *e.g*. by reaction with cyanamide. Diketones **8** can be obtained from 2-acylthiazoles **5** by acylation with acyl halides 7 (*e.g*. X = Cl) or the corresponding anhydrides. In the case where R⁶ = H, formylation of **5** will furnish corresponding keto-aldehydes **8.** Dicarbonyl compounds **8** can then be converted to enaminones **9** with appropriate bases HNR₂. If both R⁵ and R⁶ are H, then enaminones **9** can be obtained from acylthiazoles **5** directly with the aid of formamidines, amide acetals (*e.g.* dimethylformamide dimethylacetal), or aminal esters (*e.g. tert-*butoxybis(dimethylamino)methane) [13].

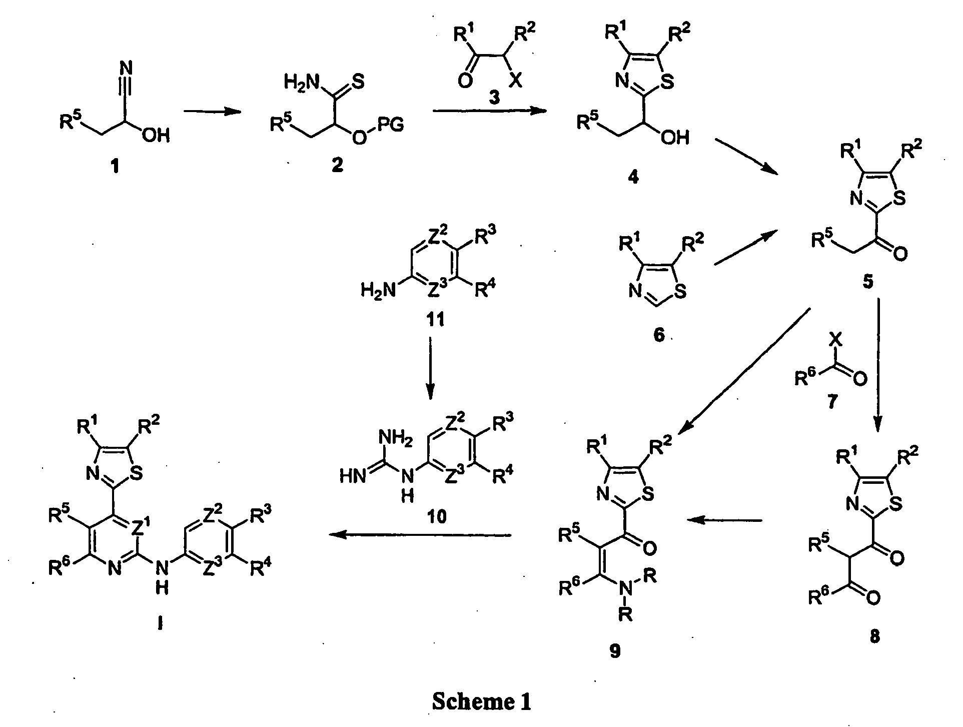

2-Acylthiazoles **5** can be prepared by lithiation of C2 in thiazoles **6,** followed by reaction of the lithiated intermediates with aldehydes R⁵CH₂CHO and oxidation of the resulting alcohols to the acylthiazoles **5.** Alternatively the lithiated thiazoles can be acylated with appropriate esters R⁵CH₂COOP, acid chlorides R⁵CH₂COCl, or acid anhydrides (R⁵CH₂CO)₂O to afford 2-acylthiazoles **5** [14,15]. It is also possible to prepare Grignard reagents from 2-unsubstituted thiazoles **6** with the aid of ethyl magnesium bromide, followed by reaction of these reagents with acid anhydrides (R⁵CH₂CO)₂O to afford 2-acylthiazoles **5** [16]. Another general route towards 2-acylthiazoles **5** [17] starts from lactonitriles 1, which can be prepared by addition of HCN to aldehydes R⁵CH₂CHO. Protection of the alcohol function, *e.g*. as the tetrahydropyran ether (PG = tetrahydropyran-2-yl), is then performed prior to conversion of the nitrile function to the thioacetamide in products **2** with *e.g*. a hydrogen sulphide-saturated solution of ethanol containing diethylamine. The thioamides **2** can then be condensed with α-haloketones **3** according to the Hantzsch thiazole synthesis [8], followed by removal of the protecting group, to afford 1-thiazol-2-yl-ethanols **4**. These are subsequently oxidized, *e.g*. with the aid of potassium dichromate in glacial acetic acid, to the ketones **5**.

Alternative synthetic routes for compounds of general structure I are shown in *Scheme* 2.

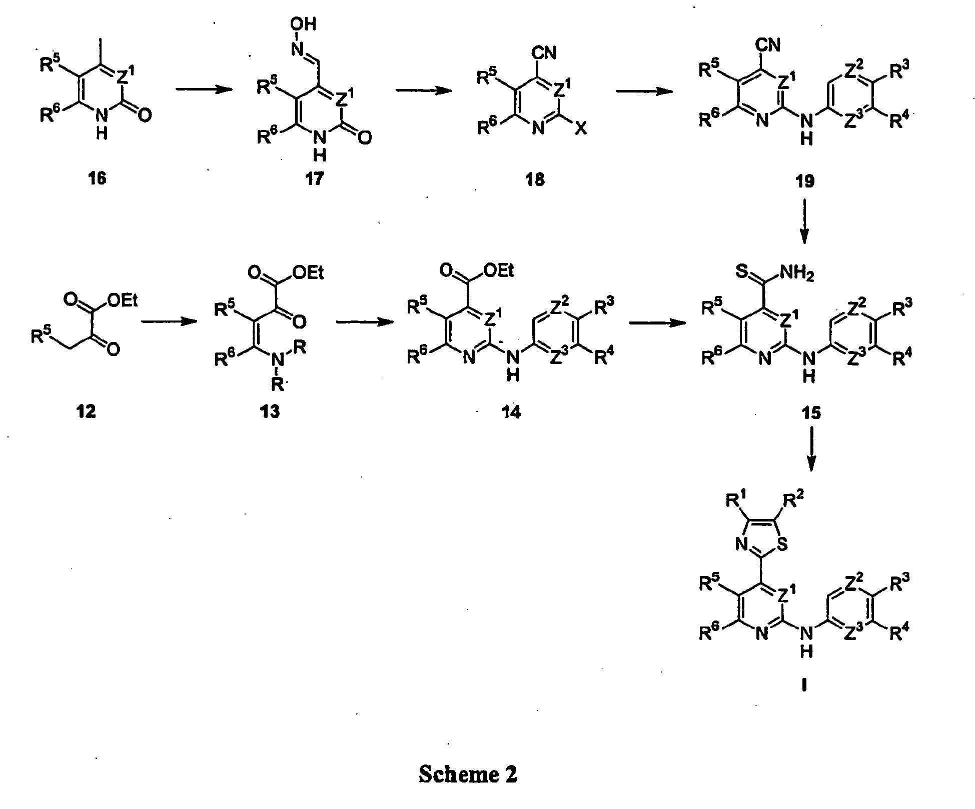

Here the enaminones **13,** derived from ethyl pyruvates **12** [18], are condensed with arylguanidines **10** [19]. The product pyrimidine esters **14** can readily be converted to the corresponding primary carboxamides, *e.g.* with ethanolic ammonia solution [20,21]. The carboxamide function is then converted to the thiocarboxamide, *e.g.* using Lawesson's reagent [22-24], phosphorus pentasulphide [18,20,21], or ammonium sulphide [25]. The same conversion can also be achieved by activation of the amide as a pyridyl triflate, followed by thiolysis with ammonium sulphide [26].

A synthetic route applicable regardless of whether **Z¹** in general structure **I** is **N** (pyrimidines) or CH (pyridines) involves 2-halogeno-4-cyano-pyrimidines (*Scheme 2;* **18**, Z¹ = N) or pyridines (**18**, Z¹ = CH) as key intermdiates. These can be prepared by many methods known in the art [27-29]. In the pyrimidine case (Z¹ = N) a convenient synthesis [30] starts from 4-methyl-1*H*-pyrimidin-2-ones 16, which are oximated to **17.** These aldoximes can be dehydrated to the corresponding nitriles and if the dehydration is carried out with *e.g.* phosphorous oxychloride, then chloronitriles **18** (X = Cl) are obtained directly [31]. The halogen group (X) in **18** can then be substituted with arylamines **11** to afford intermediates **19** [32]. The nitrile function in 19 is then oxidized to the thiocarboxamide **15,** *e.g.* by using a refluxing methanol solution containing ammonium sulphide. Finally thiazole ring formation is carried out, *e.g.* by heating a methanolic solution of **15** and suitable α-haloketones 3 in the presence of an organic base such as pyridine. These reactions proceed particularly smoothly when microwave irradiation is applied.

The present invention is further described by way of example and with reference to the following figures, wherein:
Figure 1 shows the lack of β-catenin accumulation in HEK293 cells in response to exposure to compound **XIV** (test compound) β-catenin.
Figure 2 shows the effect of compound **XIV** on oral glucose tolerance in ZDF fa/fa rats. Test compound was administrated in 10-11 weeks old ZDF rats at 5 mg/kg i.v. at -270 and -30 min. At 0 min 2g/kg oral glucose load was given and blood samples were collected at 15 min intervals to determine the blood glucose levels.

### EXAMPLES

The example compounds of the invention are listed in Table 1.

### Example 1

### General

NMR spectra were recorded using a Varian INOVA-500 instrument. Chemical shifts are reported in parts per million relative to internal tetramethylsilane standard. Mass spectra were obtained using a Waters ZQ2000 single quadrupole mass spectrometer with electrospray ionization (ESI). Analytical and preparative RP-HPLC was performed using Vydac 218TP54 (250 x 4.6 mm) and 218TP1022 (250 x 22 mm) columns, respectively. Linear gradient elution using H₂O/MeCN systems (containing 0.1 % CF₃COOH) at flow rates of 1 mL/min (analytical) and 9 mL/min (preparative) was performed. Purity was assessed by integration of chromatograms (λ = 254 nm). Silica gel (EM Kieselgel 60, 0.040-0.063 mm, Merck) or ISOLUTE pre-packed columns (Jones Chromatography Ltd. UK) were used for flash chromatography.

### Example 2

### 3-Dimethylamino-1-thiazol-2-yl-propenone

1-Thiazol-2-yl-ethanone (1.9 g, 14.9 mmol) and dimethylformamide dimethylacetal (1.98 mL, 14.9 mmol) were combined and heated at 85°C for 8 h. Following cooling and concentration, the residue was crystallized from diethyl ether and the resulting solid title product was filtered (1.56 g, 58%). ¹H-NMR (DMSO-*d₆*) δ: 2.91 & 3.19 (6H, s, N(CH₃)₂), 6.01 (1H, s, CH), 7.82 (1H, s, CH), 7.92 (1H, d, ArH, *J*= 3.4 Hz), 7.95 (1H, d, ArH, *J*= 3.4 Hz). ESI-MS: *m*/*z* 183 [M + 1]⁺; C₈H₁₀N₂OS requires 182.24. Anal RP-HPLC: t_{R} 18.4 min (0 - 60 % MeCN gradient over 20 min); purity > 95 %.

### 3-Dimethylamino-1-(4,5-dimethyl-thiazol-2-yl)-propenone

1-(4,5-Dimethyl-thiazol-2-yl)-ethanone (1.8 g, 11.8 mmol), and dimethylformamide dimethylacetal (1.7 mL, 14.2 mmol) were combined and heated at 85 °C for 8 h. Following cooling, the resulting crystalline solid was filtered and washed with cold diethyl ether (2.1 g, 85 %). ¹H-NMR (DMSO-*d₆*): δ 2.25 (3H, s, CH₃), 2.38 (3H, s, CH₃), 2.81 & 3.18 (6H, s, N(CH₃)₂), 5.91 (1H, s, CH), 7.79 (1H, s, CH). ESI-MS: *m*/*z* 210 [M + 1]⁺; C₁₀H₁₄N₂OS requires 210.08 . Anal. RP-HPLC: *t*_{R} 14.5 min (0 - 60 % MeCN gradient over 20 min); purity > 95 %.

### Example 3

### (6-Chloro-pyridin-3-yl)-(4-th iazol-2-yl-pyrimidin-2-yl)-amine (XIV)

3-Dimethyl-amino-1-thiazol-2-yl-propenone (120 mg, 0.66 mmol), N-(6-chloro-pyridin-3-yl)-guanidine nitrate (154 mg, 0.66 mmol), prepared by guanylation of 6-chloro-pyridin-3-ylamine with aqeous cyanamide solution in the presence of nitric acid, and potassium carbonate (228 mg, 1.66 mmol) were combined in 2-methoxyethanol and the mixture was heated at 120°C for 20 h. Inorganic insolubles were removed by filtration and the filtrate was concentrated. The residue was fractionated by silica gel column chromatography. Pooling of appropriate eluant fractions and removal of the solvent afforded the title compound (69 mg, 27 %). ¹H-NMR (DMSO-*d₆*) δ: 7.48 (1H, d, ArH, *J* = 8.3 Hz), 7.54 (1H, d, ArH, *J*= 4.9 Hz), 8.04 (1H, d, ArH, *J =* 3.4 Hz), 8.11 (1H, d, ArH, *J=* 3.4 Hz), 8.25 (1H, dd, ArH, *J*= 8.3, 2.9 Hz), 8.69 (1H, d, ArH, *J* = 4.9 Hz), 8.85 (1H, d, ArH, *J* = 2.9 Hz), 10.19 (1H, s, NH). ESI-MS: *m*/*z* 290 [M + H]⁺; C₁₂H₈ClN₅S requires 289.02. Anal. RP-HPLC: *t*_{R} 20.45 min (0 - 60 % MeCN gradient over 20 min); purity > 95 %.

Example compounds **Vm-XIII, XVI, XVII-XX, XXIII,** and **XXVII** listed in **T**able 1 were prepared similarly by condensation of the appropriate 3-dimethyl-amino-1-thiazol-2-yl-propenone and phenyl- or pyridyl-guanidine.

### N-[4-(4,5-Dimethyl-thiazol-2-yl)-pyrimidin-2-yl]-benzene-1,3-diamine (VIII)

¹H-NMR (DMSO-*d₆*) δ: 2.36 (3H, s, CH₃), 2.43 (3H, s, CH₃), 4.86 (2H, s, NH₂), 6.21 (1H, d, ArH, *J*= 8.7 Hz), 6.93 (1H, dd, ArH, *J*= 8.7, 8.7 Hz), 6.98 (1H, d, ArH, *J*= 8.7 Hz), 7.02 (1H, s, ArH), 7.30 (1H, d, ArH, *J =* 5.4 Hz), 8.52 (1H, d, ArH, *J =* 5.4 Hz), 9.46 (1H, s, NH). ESI-MS: *m*/*z* 298.3 [M + H]⁺; C₁₅H₁₅N₅S requires 297.10. Anal. RP-HPLC: *t*_{R} 14.61 min (0 - 60 % MeCN gradient over 20 min); purity > 95 %.

### 3-[4-(4,5-Dimethyl-thiazol-2-yl)-pyrimidin-2-ylamino]-phenol (IX)

¹H-NMR (DMSO-*d₆*) δ:2.36 (3H, s, CH₃), 2.44 (3H, s, CH₃), 6.39 (1H, d, ArH, *J*= 8.8 Hz), 7.07 (1H, dd, ArH, *J=* 8.8, 8.8 Hz), 7.36 (2H, m, ArH), 7.34 (1H, d, ArH, *J* = 5.4 Hz), 8.56 (1H, d, ArH, *J=* 5.4 Hz), 9.36 (1H, s, OH), 9.64 (1H, s, NH). ESI-MS: *m*/*z* 299.3 [M + H]⁺; C₁₅H₁₄OS requires 298.09. Anal. RP-HPLC: *t*_{R} 18.44 min (0 - 60 % MeCN gradient over 20 min); purity > 95 %.

### [4-(4,5-Dimethyl-thiazol-2-yl)-pyrimidin-2-yl]-(3-trifluoromethyl phenyl)-amine (X)

¹H-NMR (DMSO-*d₆*) δ: 2.36 (3H, s, CH₃), 2.44 (3H, s, CH₃). 7.31 (1H, d, ArH, *J* = 8.8 Hz), 7.42 (1H, d, ArH, *J=* 5.4 Hz), 7.54 (1H, d, ArH, *J*= 8.8, 8.8 Hz), 7.94 (1H, d, ArH, *J*= 8.8 Hz), 8.41 (1H, s, ArH), 8.62 (1H, d, ArH, *J=* 5.4 Hz), 10.16 (1H, s, NH). ESI-MS: *m*/*z* 351.4 [M + 1]⁺; C₁₆H₁₃F₃N₄S requires 350.08. Anal. RP-HPLC: *t*_{R} 20.07 min (20 - 80 % MeCN gradient over 20 min); purity > 95 %.

### (4-Chloro-3-trifluoromethyl-phenyl)-[4-(4,5-dimethyl thiazol-2-yl)-pyrimidin-2-yl]-amine (XI)

¹H-NMR (DMSO-*d₆*) δ: 2.36 (3H, s, CH₃), 2.43 (3H, s, CH₃), 7.46 (1H, d, ArH, *J*= 5.4 Hz), 7.65 (1H, d, ArH, *J* = 8.8 Hz), 7.98 (1H, dd, ArH, *J* = 8.8, 2.9 Hz), 8.52 (1H, d, ArH, *J=* 2.9 Hz), 8.65 (1H, d, ArH, *J=* 5.4 Hz), 10.38 (1H, s, NH). ESI-MS: *m*/*z* 385.3 [M + H]⁺; C₁₆H₁₂ClF₃N₄S requires 384.04. Anal. RP-HPLC: *t*_{R} 24.67 min (20 - 80 % MeCN gradient over 20 min); purity > 95 %.

### [4-(4,5 Dimethyl-thiazol-2-yl)-pyrimidin-2yl]-(3-nitro-phenyl)-amine (XII)

¹H-NMR (DMSO-*d₆*) δ: 2.38 (3H, s, CH₃), 2.45 (3H, s, CH₃), 7.47 (1H, d, ArH, *J*= 5.4 Hz), 7.61 (1H, dd, ArH, *J =* 8.8, 8.8 Hz), 7.84 (1H, d, ArH, *J =* 8.8 Hz), 8.08 (1H, d, ArH, *J*= 8.8 Hz), 8.67 (1H, d, ArH, *J*= 5.4 Hz), 9.98 (1H, s, ArH), 10.34 (1H, s, NH). ESI-MS: *m*/*z* 328.4 [M + H]⁺; C₁₅H₁₃N₅O₂S requires 327.08. Anal. RP-HPLC: *t*_{R} 23.70 min (10 - 70 % MeCN gradient over 20 min); purity > 95 %.

### (6-Methoxy-pyridin-3-yl)-(4-thiazol-2-yl-pyrimidin-2-yl)-amine (XIII)

¹H-NMR (DMSO-*d₆*) δ: 3.72 (3H, s, OCH₃), 6.82 (1H, d, ArH, *J*= 8.8 Hz), 7.43 (1H, d, ArH, *J=* 4.9 Hz), 7.99 (1H, d, ArH, *J=* 3.4 Hz), 8.03 (1H, dd, ArH, *J*= 8.8, 2.9 Hz), 8.08 (1H, d, ArH, *J*= 3.4 Hz), 8.56 (1H, d, ArH, *J*= 2.9 Hz), 8.60 (1H, d, ArH, *J=* 4.9 Hz), 9.76 (1H, s, NH). ESI-MS: m/z 285 [M + H]⁺; C₁₃H₁₁N₅OS requires 285.07. Anal. RP-HPLC: *t*_{R} 16.49 min (0 - 60 % MeCN gradient over 20 min); purity > 95 %.

### [4-(4,5-Dimethyl-thiazol-2-yl)-pyrimidin-2-yl]-(6-methoxy-pyridin-3-yl)-amine (XVI)

¹H-NMR (DMSO-*d₆*) δ: 2.36 (3H, s, CH₃), 2.44 (3H, s, CH₃), 3.83 (3H, s, OCH₃), 6.83 (1H, d, ArH, *J =* 8.8 Hz), 7.34 (1H, d, ArH, *J* = 5.4 Hz), 8.02 (1H, dd, ArH, *J* = 2.9 Hz), 8.55 (1H, d, ArH, *J =* 5.4 Hz), 8.56 (1H, d, ArH, *J* = 2.9 Hz), 9.70 (1H, s, NH). ESI-MS: *m*/*z* 314.32 [M + H]⁺; C₁₅H₁₅N₅OS requires 313.10. Anal. RP-HPLC: *t*_{R} 20.26 min (0 - 60 % MeCN gradient over 20 min); purity > 95 %.

### (6-Chloro-pyridin-3 yl)-[4-(4,5-dimethyl-thiazol-2-yl)-pyrimidin-2-yl]-amine (XVII)

¹H-NMR (DMSO-*d₆*) δ: 2.36 (3H, s, CH₃), 2.45 (3H, s, CH₃), 7.44 (1H, d, ArH, *J*= 5.4 Hz), 7.48 (1H, d, ArH, *J* = 8.8 Hz), 8.21 (1H, dd, ArH, *J* = 8.8, 2.9 Hz), 8.62 (1H, d, ArH, *J* = 5.4 Hz), 8.67 (1H, d, ArH, *J =* 2.9 Hz), 10.13 (1H, s, NH). ESI-MS: *m*/*z* 318.24 [M + H]⁺; C₁₄H₁₂ClN₅S requires 317.05. Anal. RP-HPLC: *t*_{R} 21.72 min (10 - 70 % MeCN gradient over 20 min); purity > 95 %.

### [4-(4,5-Dimethyl-thiazol-2-yl)-pyrimidin-2-yl]-(4-morpholin-4-yl-phenyl)-amine (XVIII)

¹H-NMR (DMSO-*d₆*) δ: 2.36 (3H, s, CH₃), 2.44 (3H, s, CH₃), 3.05 (4H, m, morpholine-H), 3.74 (4H, m, morpholine-H), 6.92 (1H, d, *J*= 8.5 Hz, ArH), 7.28 (1H, d, *J*= 5.0 Hz, pyrimidine-H), 7.65 (1H, d, *J* = 8.5 Hz, ArH), 8.51 (1H, d, *J* = 5.0 Hz, pyrimidine-H), 9.54 (1 H, s, NH). ESI-MS: *m*/*z* 368.5 [M + H]⁺; C₁₉H₂₁N₅OS requires 367.15. Anal. RP-HPLC: *t*_{R} 13.77 min (10 - 70 % MeCN gradient over 20 min); purity > 95 %.

### [4-(4,5-Dimethyl-thiazol-2-yl)-pyrimidin-2-yl]-(4-methyl-3-nitro-phenyl)-amine (XIX)

¹H-NMR (CDCl₃) δ: 2.43 (3H, s, CH₃), 2.47 (3H, s, CH₃), 2.58 (3H, s, CH₃), 7.29 (1H, d, *J*= 8.0 Hz, ArH), 7.35 (1H, s, NH), 7.52 (1H, d, *J*= 5.5 Hz, pyrimidine-H), 7.59 (1 H, dd, *J =* 8.0, 2.5 Hz, ArH), 8.52 (1H, d, *J =* 5.5 Hz, pyrimidine-H), 8.72 (1H, d, *J =* 2.5 Hz, ArH). ESI-MS; *m*/*z* 342.4 [M + H]⁺; C₁₆H₁₅N₅O₂S requires 341.09. Anal. RP-HPLC: *t*_{R} 10.49 min (10 - 70 % MeCN gradient over 20 min); purity > 95 %.

### 4-[4-(4,5-Dimethyl-thiazol-2-yl)-pyrimidin-2-yl-amino]-phenol (XX)

¹H-NMR (CDCl₃) δ: 2.40 (3H, s, CH₃), 2.43 (3H, s, CH₃), 6.84 (1H, m, ArH), 7.37 (1H, d, *J* = 5.0 Hz, pyrimidine-H), 7.47 (1H, m, ArH), 8.40 (1H, d, *J* = 5.0 Hz, pyrimidine-H). ESI-MS: *m*/*z* 299.4 [M + H]⁺; C₁₅H₁₄N₄OS requires 298.09. Anal. RP-HPLC: *t*_{R} 13.42 min (10 - 70 % MeCN gradient over 20 min); purity > 95 %.

### (6-Pyrrolidin-1-yl-pyridin-3-yl)-(4-thiazol-2-yl-pyrimidin-2-yl)-amine (XXIII)

¹H-NMR (DMSO-*d₆*) δ: 1.80 (2H, m, CH₂), 1.94 (2H, m, CH₂), 3.05 (2H, m, NCH₂), 3.36 (2H, m, NCH₂), 6.44 (1H, d, ArH, *J* = 8.8 Hz), 7.34 (1H, d, ArH, *J =* 5.4 Hz), 7.84 (1H, d, ArH, *J* = 8.8 Hz), 7.98 (1H, d, ArH, *J =* 3.4 Hz), 8.06 (1H, d, ArH, *J=* 3.4 Hz), 8.38 (1H, s, ArH), 8.54 (1H, d, ArH, *J* = 5.4 Hz), 9.45 (1H, s, NH). ESI-MS: *m*/*z* 325.41 [M + H]⁺; C₁₆H₁₆N₆S requires 324.40. Anal. RP-HPLC: *t*_{R} 14.80 min (0 - 60 % MeCN gradient over 20 min); purity > 95 %.

### (6-Chloro-5-methyl-pyridin-3-yl)-(4-thiazol-2-yl-pyrimidin-2-yl)-amine (XXVII)

¹H-NMR (DMSO-*d₆*) δ: 2.36 (3H, s, CH₃), 7.54 (1H, d, ArH, *J*= 4.9 Hz), 8.05 (1H, d, ArH, *J* = 2.9 Hz), 8.11 (1H, d, ArH, *J* = 2.9 Hz), 8.29 (1H, d, ArH, *J* = 2.9 Hz), 8.65 (1H, d, ArH, *J*= 2.9 Hz), 8.71 (1H, d, ArH, *J*= 4.9 Hz), 10.16 (1H, s, NH). ESI-MS: *m*/*z* 304.37 [M + H]⁺; C₁₃H₁₀ClN₅S requires 303.77. Anal. RP-HPLC: *t*_{R} 23.19 min (0 - 60 % MeCN gradient over 20 min); purity > 95 %.

### Example 4

### 2-Oxo-1,2-dihydro-pyrimidine-4-carbaldehyde oxime

To a solution of 4-methyl-1H-pyrimidin-2-one hydrochloride (14.7 g, 0.1 mol) in 50 % aqueous acetic acid (100 mL) at 15°C, was added in one portion sodium nitrite (10.4 g, 0.15 mol) with vigorous stirring. After an exothermic reaction (~ 40°C) a yellow precipitate formed. This was filtered, washed with cold water, and dried under vacuum to afford the title compound (13.51 g, 97 %). ¹H-NMR (DMSO-*d₆*) δ: 6.65 (1H, d, ArH, *J*= 6.4 Hz), 7.75 (1H, s, CH), 7.91 (2H, d, ArH, *J* = 6.4 Hz), 11.87 (1H, s, NH), 12.41 (1H, s, OH). ESI-MS: *m*/*z* 139.89 [M + H]⁺; C₅H₅N₃O₂ requires 139.11. Anal. RP-HPLC: *t*_{R} 5.35 min (0 - 60 % MeCN gradient over 20 min); purity > 95 %.

### 2-Chloro-pyrimidine-4-carbonitrile

A mixture of 2-oxo-1,2-dihydro-pyrimidine-4-carbaldehyde oxime (5 g, 0.036 mol) in cold phosphorous oxychloride (20 mL) was warmed slowly until a vigorous reaction commenced, at which time warming was discontinued. Once complete dissolution had taken place, diethyl-phenyl-amine (2.5 mL) was added and the reaction mixture was refluxed for a further 30 min. After cooling the mixture was poured over ~150 g of ice and was extracted into dichloromethane (5 × 30 mL), then washed with saturated sodium bicarbonate solution (2 × 50 mL) and water (2 × 50mL), before drying over anhydrous magnesium sulphate. After removal of solvent, the residue was dried under vacuum and solidified upon standing. No further purification was necessary. ¹H-NMR (DMSO-*d₆*) δ: 7.63 (1H, d, ArH, *J=* 4.9 Hz), 8.89 (1H, d, ArH, *J=* 4.9 Hz). Anal. RP-HPLC: *t*_{R} 12.07 min (0 - 60 % MeCN gradient over 20 min); purity > 95 %.min.

### 2-(4-Chloro-phenylamino)-pyrimidine-4-carbonitrile

2-Chloro-pyrimidine-4-carbonitrile (1.03 g, 7.38 mmol) and 4-chloroaniline (0.94 g, 7.38 mmol) were dissolved in ethanol (10 mL) and the solution was heated at 100 °C for 90 min. Upon cooling the title product crystallized from the reaction mixture and was filtered (0.84 g, 42 %). ¹H-NMR (DMSO-*d₆*) δ: 7.37 (2H, d, ArH, *J*= 8.8 Hz), 7.42 (1H, d, ArH, *J*= 4.9 Hz), 7.72 (2H, d, ArH, *J*= 8.8 Hz), 8.78 (1H, d, ArH, *J*= 4.9 Hz), 10.32 (1H, s, NH). ESI-MS: *m*/*z* 231.16 [M + H]⁺; C₁₁H₇ClN₄ requires 230.65. Anal. RP-HPLC: *t*_{R} 22.41 min (0 - 60 % MeCN gradient over 20 min); purity > 95 %.

### 2-(4-Chloro-phenylamino)-pyrimidine-4-carbothioic acid amid

A solution of 2-(4-chloro-phenylamino)-pyrimidine-4-carbonitrile (463 mg, 2.01 mmol) and ammonium sulphide (20 % w/w in H₂O, 4 mL) in methanol (10 mL) was heated under reflux for 5 h. Upon cooling water (1 mL) was added and the resulting precipitate was filtered to afford the title compound (369 mg, 69 %). ¹H-NMR (DMSO-*d₆*) δ: 7.44 (2H, d, ArH, *J*= 8.8 Hz), 7.53 (1H, d, ArH, *J*= 4.9 Hz), 7.78 (2H, d, ArH, *J* = 8.8 Hz), 8.64 (1H, d, ArH, *J* = 4.9 Hz), 9.61 & 10.39 (2H, s, S=CNH₂), 9.92 (1H, s, NH). ESI-MS: *m*/*z* 265.81 [M + H]⁺; C₁₁H₉ClN₄S requires 264.73. Anal. RP-HPLC: *t*_{R} 22.03 min (0 - 60 % MeCN gradient over 20 min); purity > 95 %.

### 2-(6-Chloro-pyridin-3-ylamino)-pyrimidine-4-carbonitrile

This compound was prepared from 2-chloro-pyrimidine-4-carbonitrile and 6-chloro-pyridin-3-ylamine. ¹H-NMR (DMSO-*d₆*) δ: 7.48 (1H, d, ArH, *J*= 4.9 Hz), 7.51 (1H, d, ArH, *J* = 8.8 Hz), 8.17 (1H, dd, ArH, *J* = 8.8, 2.9 Hz), 8.70 (1H, d, ArH, *J* = 2.9 Hz), 8.83 (1H, d, ArH, *J*= 4.9 Hz), 10.51 (1H, s, NH). ESI-MS: *m*/*z* 231 [M]⁺; C₁₀H₆ClN₅ requires 231.03. Anal. RP-HPLC: *t*_{R} 17.84 min (0 - 60 % MeCN gradient over 20 min); purity > 95 %.

### 2-(6-Chloro-pyridin-3-ylamino)-pyrimidine-4-carbothioic acid amide

This compound was prepared from 2-(6-chloro-pyridin-3-ylamino)-pyrimidine-4-carbonitrile with ammonium sulphide in an analogous manner as described above for 2-(4-chloro-phenylamino)-pyrimidine-4-carbothioic acid amide. ¹H-NMR (DMSO-*d₆*): 7.36 (1H, d, ArH, *J =* 4.9 Hz), 7.45 (1H, d, ArH, *J =* 8.8 Hz), 7.83 & 7.94 (2H, s, S=CNH₂), 8.28 (1H, dd, ArH, *J*= 8.8, 2.9 Hz), 8.74 (2H, m, ArH), 10.16 (1H, s, NH). ESI-MS: *m*/*z* 265 [M + H]⁺ (~ 20 %); C₁₀H₈ClN₅S requires 265.02. Anal. RP-HPLC: *t*_{R} 13.94 min (0 - 60 % MeCN gradient over 20 min); purity > 95 %.

### Example 5

### 1-{2-[2-(4-Chloro-phenylamino)-pyrimidin-4-yl]-4-methyl-thiazol-5-yl}-ethanone (II)

A mixture of 2-(4-chloro-phenylamino)-pyrimidine-4-carbothioic acid amide (31 mg, 0.113 mmol), 3-chloro-pentane-2,4-dione (30 µL, 0.249 mmol), and pyridine (14 µL, 0.17 mmol) in methanol (2 mL) was heated at 150°C in a Smith Creator microwave reactor (Personal Chemistry AB, Uppsala, Sweden) for 15 min. Upon cooling the resulting precipitate of title compound was collected by filtration and was washed with cold methanol (21 mg, 54 %). ¹H-NMR (DMSO-*d₆*): 2.63 (3H, s, CH₃), 2.74 (3H, s, C=OCH₃), 7.39 (2H, d, ArH, *J* = 8.8 Hz), 7.15 (1H, d, ArH, *J* = 4.9 Hz), 7.82 (2H, d, ArH, *J =* 8.8 Hz), 8.71 (1H, d, ArH, *J =* 4.9 Hz), 10.08 (1H, s, NH). ESI-MS: *m*/*z* 345 [M + H]⁺; C₁₆H₁₃ClN4OS requires 344.05. Anal. RP-HPLC: *t*_{R} 24.34 min (10 - 70 % MeCN gradient over 20 min); purity > 95 %.

Example compounds **III-VII, XV, XXII, and XXIV-XXVI** listed in **Table 1** were prepared similarly by reaction of 2-(4-chloro-phenylamino)-pyrimidine-4-carbothioic acid amide or 2-(6-chloro-pyridin-3-ylamino)-pyrimidine-4-carbothioic acid amide with the appropriate haloacyl compound (1-chloro-propan-2-one, 2-bromo-1-phenyl-ethanone, 2-chloro-3-oxo-butyric acid ethyl ester, 4-chloro-3-oxo-butyric acid methyl ester, 2-bromo-malonic acid diethyl ester, 2-bromo-propionic acid ethyl ester, or 2-bromo-4-hydroxy-butyric acid ethyl ester).

### (4-Chloro-phenyl)-[4-(4-methyl-thiazol-2-y/)-pyrimidin-2-yl]-amine (III)

¹H-NMR (DMSO-*d₆*): 2.47 (3H, s, CH₃), 7.36 (2H, d, ArH, *J* = 8.8 Hz), 7.44 (1H, d, ArH, *J*= 4.9 Hz), 7.59 (1H, s, ArH), 7.84 (2H, d, ArH, *J*= 8.8 Hz), 8.64 (1H, d, ArH, *J* = 4.9 Hz), 9.98 (1H, s, NH). ESI-MS: *m*/*z* 303 [M + H]⁺; C₁₄H₁₁ClN₄S requires 302.04. Anal. RP-HPLC: *t*_{R} 20.74 min (20 - 80 % MeCN gradient over 20 min); purity > 95 %.

### 2-[2-(4-Chloro-phenylamino)-pyrimidin-4-yl]-4-hydroxy-thiazole-5-carboxylic acid ethyl ester (VII)

¹H-NMR (DMSO-*d₆*): 1.28 (3H, t, CH₃, *J* = 7.3 Hz), 4.24 (2H, q, CH₂, *J =* 7.3 Hz), 7.31 (1H, d, ArH, *J*= 5.4 Hz), 7.36 (1H, d, ArH, *J*= 8.8 Hz), 7.78 (1H, d, ArH, *J* = 8.8 Hz), 8.70 (1H, d, ArH, *J =* 5.4 Hz), 9.94 (1H, s, NH), 12.18 (1H, s, OH). ESI-MS: *m*/*z* 377.25 [M + H]⁺; C₁₆H₁₃ClN₄O₃S requires 376.04. Anal. RP-HPLC: *t*_{R} 20.90 min (20 - 80 % MeCN gradient over 20 min); purity > 95 %.

### 1-{2-[2-(6-Chloro-pyridin-3-ylamino)-pyrimidin-4-yl]-4-methyl-thiazol-5-yl}-ethanone (XV)

¹H-NMR (DMSO-*d₆*): 2.63 (3H, s, CH₃), 2.74 (3H, s, C=OCH₃), 7.52 (1H, d, ArH, *J*= 8.8 Hz), 7.57 (1H, d, ArH, *J =* 5.4 Hz), 8.22 (1H, dd, ArH, *J=* 8.8, 2.9 Hz), 8.75 (1H, d, ArH, *J=* 5.4 Hz), 8.86 (1H, d, ArH, *J=* 2.9 Hz), 10.28 (1H, s, NH). ESI-MS: *m*/*z* 346 [M + H]⁺; C₁₅H₁₂ClN₅OS requires 345.05. Anal. RP-HPLC: *t*_{R} 19.77 min (10 - 70 % MeCN gradient over 20 min); purity > 95 %.

### 2-[2-(4-Chloro-phenylamino)-pyridin-4-yl]-5-methyl-thiazol-4-ol (XXII)

¹H-NMR (DMSO-*d₆*): 2.28 (3H, s, CH₃), 7.26 (1H, d, ArH, *J* = 5.4 Hz), 7.37 (2H, d, ArH, *J =* 8.8 Hz), 7.36 (2H, d, ArH, *J =* 8.8 Hz), 7.82 (1H, d, ArH, *J* = 5.4 Hz), 9.91 (1H, s, NH), 10.61 (1H, s, OH). ESI-MS: *m*/*z* 319.24 [M + H]⁺; C₁₅H₁₂ClN₃OS requires 317.04. Anal. RP-HPLC: *t*_{R} 16.76 min (20 - 80 % MeCN gradient over 20 min); purity > 95 %.

### 2-[2-(6-Chloro-pyridin-3-ylamino)-pyrimidin-4-yl]-4-hydroxy-thiazole-5-carboxylic acid ethyl ester (XXIV)

¹H-NMR (DMSO-*d₆*): 1.28 (3H, t, CH₃, *J* = 6.8 Hz), 4.25 (2H, q, OCH₂, *J* = 6.8 Hz), 7.43 (1H, d, ArH, *J =* 4.9 Hz), 7.50 (1H, d, ArH, *J =* 8.8 Hz), 8.18 (1H, dd, ArH, *J* = 8.8, 2.9 Hz), 8.75 (1H, d, ArH, *J*= 4.9 Hz), 8.88 (1H, d, ArH, *J*= 2.9 Hz), 10.25 (1H, s, NH). ESI-MS: *m*/*z* 378.42 [M + H]⁺; C₁₅H₁₂ClN₅O₃S requires 377.81. Anal. RP-HPLC: *t*_{R} 14.67 min (10 - 70 % MeCN gradient over 20 min); purity > 95 %.

### 2-[2-(6-Chloro-pyridin-3-ylamino)-pyrimidin-4-yl]-5-methyl-thiazol-4-ol (XXV)

¹H-NMR (DMSO-*d₆*): 2.28 (3H, s, CH₃), 7.32 (1H, d, ArH, *J* = 4.9 Hz), 7.47 (1H, d, ArH, *J =* 8:8 Hz), 8.22 (1H, dd, ArH, *J =* 8.8, 2.9 Hz), 8.62 (1H, d, ArH, *J =* 4.9 Hz), 8.86 (1H, d, ArH, *J*= 2.9 Hz), 10.10 (1H, s, NH), 10.64 (1H, s, OH). ESI-MS: *m*/*z* 320.22 [M + H]⁺; C₁₃H₁₀ClN₅OS requires 319.77. Anal. RP-HPLC: *t*_{R} 15.51 min (10 - 70 % MeCN gradient over 20 min); purity > 95 %.

### 2-[2-(6-Chloro-pyridin-3-ylamino)-pyrimidin-4-yl]-5-(2-hydroxy-ethyl)-thiazol-4-ol (XXVI)

¹H-NMR (DMSO-*d₆*): 2.83 (2H, t, CH₂, *J =* 6.7 Hz), 3.35 (CH₂OH, *J =* 6.7 Hz), 4.93 (1H, s, OH), 7.33 (1H, d, ArH, *J*= 5.4 Hz), 7.47 (1H, d, ArH, *J*= 8.3 Hz), 8.22 (1H, dd, ArH, *J* = 8.3, 2.4 Hz), 8.61 (1H, d, ArH, *J* = 5.4 Hz), 8.88 (1H, d, ArH, *J* = 2.4 Hz), 10.10 (1H, s, NH), 10.66 (1H, s, OH). ESI-MS: m/z 348.34 [M + H]⁺; C₁₄H₁₂ClN₅O₂S requires 349.80. Anal. RP-HPLC: *t*_{R} 9.41 min (20 - 80 % MeCN gradient over 20 min); purity > 95 %.

### Example 6

### 2-(3-Hydroxy phenylamino)-isonicotinonitrile

2-Chloro-isonicotinonitrile (1.0 eq) was dissolved in anhydrous toluene before addition of 3-amino-phenol (1.1 eq), palladium-II acetate (0.1 eq), and bis(diphenylphosphino)propane (0.12 eq). The reaction mixture was stirred at room temperature for 10 min before addition of sodium *tert*-butoxide (1.3 eq). The resulting suspension was heated at 70°C) for 20 h. The reaction mixture was cooled, diluted with diethyl ether, and washed with brine. The organic fraction was dried (MgSO₄) and concentrated under vacuum. The crude product was purified silica gel column chromatography [heptane : ethyl acetate (12:1 → 1:1)] to afford the desired title product, as well as 2-chloro-*N*-(3-hydroxy-phenyl)-isonicotinamidine as a side product [33].

### 2-(3-Hydroxy-phenylamino)-thioisonicotinamide

2-(3-Hydroxy-phenylamino)-isonicotinonitrile was dissolved in methanol before addition of ammonium sulfide (20 %, in water). The reaction mixture was heated at 75 °C) for 3 h. Water was added to the cooling solution and the desired title product was filtered, washed with cold water, and dried.

### 1-{2-[2-(4-Hydroxy-phenylamino)-pyridin-4-yl]-4-methyl-thiazol-5-yl}-ethanone (XXI)

2-(3-Hydroxy-phenylamino)-thioisonicotinamide (1.0 eq) was dissolved in methanol before the addition of pyridine (1.4 eq) and 3-chloro-2,4-pentadione (1.1 eq). The reaction mixture was heated (150 °C) in a Smith Creator microwave reactor for 15 min. The resulting solution was cooled and concentrated under vacuum to obtain crude product. The crude product was purified using silica gel column chromatography [heptane : ethyl acetate (12:1 → 3:1)] to afford the title compound. ¹H-NMR (DMSO-*d₆)* δ: 2.58 (3H, s, CH₃), 2.71 (3H, s, CH₃), 6.70 (2H, d, ArH, *J=* 8.5 Hz), 7.15 (1H, d, ArH, *J=* 5.5 Hz), 7.36 (3H, m, ArH,), 8.14 (1H, d, Ar-H, *J=* 5.5 Hz), 9.21 (1H, s, NH). ESI-MS: *m*/*z* 326 [M + H]⁺; C₁₇H₁₅N₃O₂S requires 325.08. Anal. RP-HPLC: *t*_{R} 10.49 min (10 - 70 % MeCN gradient over 20 min); purity > 95 %.

### Example 7

### Production of recombinant proteins

CDK4/cyclin D1, CDK1/cyclin B, CDK2/cyclin E, CDK2/cyclin A, CDK9/cyclin T1 and CDK7/cyclin H, all with a His₆ tag on the N-terminus, were expressed in Sf9 insect cells using an appropriate baculovirus construct. Sf9 culture (1.6 x 10⁶ cells/mL) was infected (MOI of 3) for two days. The cells were harvested by low speed centrifugation and the protein was purified from the insect cell pellet by metal affinity chromatography. In short: the insect cell pellet was lysed in Buffer A (10 mM Tris-HCl pH 8.0, 150 mM NaCl, 0.02 % NP-40, 5 mM β-mercaptoethanol, 20 mM NaF, 1 mM Na₃VO₄, and Sigma Protease Inhibitor Cocktail) by sonication. The soluble fraction was cleared by centrifugation and loaded onto Ni-NTA-Agarose (Qiagen). Non-bound protein was washed off with 300 mM NaCl, 5 -15 mM imidazole in buffer A and the bound protein was eluted with buffer A supplemented with 250 mM imidazole. The purified proteins were extensively dialyzed against storage buffer (20 mM HEPES pH 7.4, 50 mM NaCl, 2 mM DTT, 1 mM EDTA, 1 mM EGTA, 0.02 % NP-40, 10 % v/v glycerol) and stored at -70 °C.

### Example 8

### GSK-3β kinase assay

GSK-3 was obtained from New England Biolabs (UK) Ltd., Hitchin, Herts. The recombinant enzyme was isolated from a strain of *E*. *coli* that carries a clone expressing GSK-3β derived from a rabbit skeletal muscle cDNA library [34]. Inhibition of GSK-3 function was assessed by measurement of phosphorylation of CREB phosphopeptide KRREILSRRPpSYR in the presence of test compounds. Using a 96-well assay format, GSK3 (7.5 U) was incubated for 30 min at 30 °C in a total volume of 25 µL in 20 mM MOPS pH 7.2, 25 mM β-glycerophosphate, 5 mM EGTA, 1, mM DTT, 1 mM Na₃VO₃, 40 µM CREB peptide, 15 mM MgCl₂ and 100 µM ATP (containing 0.25 µCi [γ-³²P]-ATP) in the presence of varying concentrations of test compound. The samples were transferred to 96-well p81 filter plates (Whatman Polyfiltronics, Kent, UK), and the plates were washed 4 times with 200 µL/well of 75 mM aq orthophosphoric acid. Scintillation liquid (50 µL) was added to each well, and incorporated radioactivity for each sample was determined using a scintillation counter (TopCount, Packard Instruments, Pangbourne, Berks, UK).

### CDK/cyclin kinase assays

Compounds were investigated for their CDK2/cyclin E, CDK2/cyclin A, CDK1/cyclin B, and CDK4/cyclin D1 inhibitory activity. His₆-tagged recombinant human cyclin-dependent kinases CDK1/cyclin B1, CDK2/cyclin E, CDK2/cyclin A, and CDK4 were expressed in sf9 insect cells using a baculovirus expression system. Recombinant cyclin D1 was expressed in *E*. *coli.* Proteins were purified by metal chelate affinity chromatography to greater than 90 % homogeneity. Kinase assays were performed in 96-well plates using recombinant CDK/cyclins. Assays were performed in assay buffer (25 mM β-glycerophosphate, 20 mM MOPS, 5 mM EGTA, 1 mM DTT, 1 mM Na₃VO₃, pH 7.4), into which were added 2 - 4 µg of active enzyme with appropriate substrates (purified histone H1 for CDK1 and CDK2, recombinant GST-retinoblastoma protein (residues 773-928) for CDK4). The reaction was initiated by addition of Mg/ATP mix (15 mM MgCl₂ + 100 µM ATP with 30-50 kBq per well of [γ³²P]-ATP) and mixtures incubated for 10 - 45 min, as required, at 30°C. Reactions were stopped on ice, followed by filtration through p81 or GF/C filterplates (for CDK4) (Whatman Polyfiltronics, Kent, UK). After washing 3 times with 75 mM aq orthophosphoric acid, plates were dried, scintillant added and incorporated radioactivity measured in a scintillation counter (TopCount, Packard Instruments, Pangbourne, Berks, UK). Compounds for kinase assays were made up as 10 mM stocks in DMSO and diluted into 10 % DMSO in assay buffer. Data was analysed using curve-fitting software (GraphPad Prism version 3.00 for Windows, GraphPad Software, San Diego California USA) to determine IC₅₀ values (concentration of test compound which inhibits kinase activity by 50%).

### Example 9

### Differentiation of L6 rat myocytes and 3T3 mouse adipocytes

Rat skeletal muscle myoblasts L6/G8.C5 were seeded at 2.4 x 10⁵ cells per 10 cm dish in DMEM 10 % foetal calf serum (PCS), containing penicillin/streptomycin. When 90 % confluence was reached the medium was exchanged with a MEM, supplemented with 2 %

FCS and penicillin/streptomycin. Medium was refreshed every 48 hours and 4 - 7 days later the myocytes were formed.

Mouse pre-adipocytes 3T3-F442A were seeded at 9 x 10⁵ cells per 10 cm dish in DMEM 10 % FCS, containing penicillin/streptomycin. When 90 % confluent, the same medium was supplemented with 1 µg/mL insulin. After 3-5 days (when most cells were differentiated) insulin was removed and 4 days later the cells were ready to use.

### Glycogen synthase (GS) assay

Cells on 10 cm dishes (Human Embryonic Kidney (HEK) 293 cells, L6 rat myocytes, or 3T3 mouse adipocytes) were treated with different concentrations of GSK3-inhibitors or DMSO vehicle for 90 min. Incubation medium was removed and cells were washed with ice-cold phosphate-buffered saline (PBS) prior to lysis on ice in 50 mM HEPES, pH 7.5, 10 mM EDTA, 100 mM NaF, 5 mM DTT, protease inhibitor cocktail (Sigma). After a freeze/thaw cycle the samples are sonicated for 10 sec and centrifuged at 15,000 g for 10 min at 4°C. Lysate supernatants were snap-frozen on liquid nitrogen and stored at -80°C. Lysates were assayed for glycogen synthase activity in buffer (50 mM Tris-HCl, pH 7.8, 20 mM EDTA, 25 mM NaF, 5 mM DTT, 1% glycogen, 0.3 mM UDP-glucose and 0.06 µCi of [¹⁴C]-UDP-glucose in the presence of 0.1 or 10 mM glucose-6-phosphate. The reaction was carried out for 30 min at 30°C. 70 µL of the reaction mixture (total volume 90 µL) were transferred to a GFC 96-well filter plate (bottom sealed with foil), containing 140 µL 96 % ethanol. The GFC plate was incubated for 1 h on ice and than washed with 66 % ethanol. To each well 100 µL scintillant liquid was added and the radioactivity of the samples was measured using a scintillation counter (Topcount, HP). Data are expressed as -fold increase in glycogen synthase activity ratios over those of control samples.

### Example 10

**Table 2** summarizes the biological activity of the exemplified compounds.

### Example 11

Intrinsic inhibition constants (Kᵢ) for example compound **XIV** against a number of Ser/Thr kinases were determined and are summarized in **Table 3**.

### Example 12

Example compound **XIV** increased the activity of glycogen synthase in HEK293, rat myocyte, and mouse adipocyte cells, measured by the fractional velocity of the enzyme (the ratio between the activity at 0.1 and 10 mM glucose-6-phosphate). An example of the activation in HEK293 cells and adipocyte is shown in **Table 4.**

Example compound **XIV** increased GS activity in HEK293 cells and 3T3 adipocytes. 5 µM **XIV** induced activation of GS in HEK293 cells comparable to that induced by 40 mM LiCl. In 3T3 adipocytes the activation induced by 5 µM **XIV** was approximately 2-fold higher that that induced by 40 mM LiCl.

The EC₅₀ values for **XIV**-induced activation of glycogen synthase in the three cell lines evaluated were calculated from dose-response curves: EC₅₀ (HEK293) = 1.5 ± 0.6 µM; EC₅₀ (L6 myocytes) = 4.0 ± 1.5 µM; EC₅₀ (3T3 adipocytes) = 5.0 ± 2.3 µM.

### Example 13

### Protein kinase panel selectivity screen of compound XIV

The names of the 29 kinases comprising the selectivity screen, as well as the ATP concentrations used in each kinase case, are given in **Table 5.** The kinase assays were carried out as described previously [35,36].

Compound **XIV** at a concentration of 1 µM was screened in the 29-kinase panel (Dundee University) and the results are shown bellow in **Table 6.**

Compound **XIV** was highly selective for GSK3. At the concentration used only two other kinases were slightly inhibited (about 40 %) - JNK and SAPK4.

### Example 14

### β-Catenin accumulation and transcriptional activation

One of the possible toxicities related to GSK3 inhibition is the accumulation of β-catenin, which has been implicated in the development of colon cancer [37] and melanoma [38]. The effect of example compound **XIV** on the endogenous levels of β-catenin in HEK293 cells was studied at concentrations that are effective in activation of cellular glycogen synthase. Compound **XIV** (up to 5 µM) did not change significantly the levels of cellular β-catenin, did not inhibit the phosphorylation at the GSK3-specific sites S33,37/T41, as shown in Figure 1.

Furthermore, the effect of compound **XIV** on the β-catenin-dependent transcriptional activity, as measured in a luciferase based reporter gene assay, was studied. No induction of β-catenin transcriptional activity was observed in HEK293 cells treated with compound **XIV** (up to 10 µM). At the same time, LiCl induced massive induction of β-catenin-dependent luciferase activity. These results suggest that at concentrations required for the activation of glycogen synthase compound **XIV** does not inhibit the phosphorylation of β-catenin and therefore does not induce accumulation of the protein or its transcriptional activity.

### β-Catenin-LEF/TCF regulated reporter gene assay

HEK293 cells were transfected with either β-catenin-LEF/TCF-sensitive or β-catenin-LEF/TCF-insensitive reporter vector (Upstate Biotechnology, Inc.) using Lipofectamine Plus reagent (GibcoBRL) according to the manufacturer's instructions. The next day, cells were trypsinized, washed into serum-free medium, counted and seeded at 40,000 cells per well in a 96-well plate. Subsequent to cell attachment, LiCl or GSK-3 inhibitor compound were added to the medium to the required concentrations. Control cells were DMSO vehicle treated. 16 h after inhibitor addition, cells were analyzed for luciferase activity using the Steady-Glo Luciferase assay system (Promega) according to the manufacturer's instructions.

### Example 15

### Oral glucose tolerance test (OGTT)

For the OGTT male ZDF fa/fa rats (Charles River, USA), 10-11 weeks old, were used. After 15 h fasting the animals were dosed intravenously with 5 mg/kg test compound in dosing vehicle, or with dosing vehicle (10 % DMSO, 90 % PEG-400) only at -270 and -30 min. At 0 min the rats were given 2 g/kg glucose by oral gavage. Plasma samples were taken before and every 15 min after the OGTT for determination of blood glucose.

Compound **XIV** improved the glucose tolerance in ZDF rats significantly. It decreased the reactive and absolute AUC levels by 44 and 29 %, respectively.

Various modifications and variations of the described aspects of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the relevant fields are intended to be within the scope of the following claims.

### REFERENCES

[1] Chen, Y.H.; Hansen, L.; Chen, M.X.; Bjorbaek, C.; Vestergaard, H.; Hansen, T.; Cohen, P.T.; Pedersen, O. Diabetes,1994, 43, 1234.
[2] Nikoulina, S.E.; Ciaraldi, T.P.; Mudaliar, S.; Mohideen, P.; Carter, L.; Henry, R.R. Diabetes, 2000, 49, 263.
[3] Frame, S.; Cohen, P. Biochem. J., 2001, 359, 1.
[4] Mattson, M.P. Nat Rev Mol Cell Biol, 2000, 1, 120.
[5] Goedert, M. Curr. Opin. Gen. Dev., 2001, 11, 343.
[6] Zhu, A.J.; Watt, F.M. Development, 1999, 126, 2285*.*
[7] DasGupta, R.; Fuchs, E. Development,1999, 126, 4557.
[8] Katritzky, A.R; Ostercamp, D.L.; Yousaf, T.I. Tetrahedron, 1987, 43, 5171.
[9] Baum, F. Chem. Ber., 1908, 41, 532.
[10] Zimmermann, J.; Caravatti, G.; Mett, H.; Meyer, T.; Müller, M.; Lydon, N.B.; Fabbro, D. Arch. Pharm. Pharm. Med. Chem., 1996, 329, 371.
[11] Feichtinger, K.; Zapf, C.; Sings, H.L.; Goodman, M. J. Org. Chem., 1998, 63, 3804.
[12] Katritzky, A.R.; Parris, RL.; Allin, S.M.; Steel, P.J. Synth. Commun.,1995, 25, 1173.
[13] Bredereck, H.; Effenberger, F.; Botsch, H. Chem. Ber., 1964, 97, 3397.
[14] Davies, D.H.; Hall, J.; Smith, E.H. J. Chem. Soc. Perkin Trans. 1, 1991, 2691.
[15] Jones, G.; Ollivierre, H.; Fuller, L.S.; Young, J.H. Tetrahedron, 1991, 47, 2861
[16] Metzger, J.; Koether, B. Bull. Soc. Chim. France,1953, 702.
[17] Kiss, J.; D'Souza, R; Spiegelberg, H. Helv. Chim. Acta, 1968, 51, 825.
[18] Riley, T.A.; Hennen, W.J.; Dalley, N.K.; Wilson, B.E.; Robins, RK.; Larson, S.B. J. Heterocycl. Chem., 1987, 24, 955
[19] Dorigo, P.; Fraccarollo, D.; Santostasi, G.; Maragno, I.; Floreani, M.; Borea, P.A.; Mosti, L.; Sansebastiano, L.; Fossa, P.; et al. J Med. Chem., 1996, 39, 3671
[20] Goel, O.P.; Krolls, U. Synthesis, 1987, 162.
[21] Raucher, S.; Klein, P. J. Org. Chem., 1981, 46, 3558.
[22] Yde, B.; Yousif, N.M.; Pedersen, U.; Thomsen, I.; Lawesson, S.O. Tetrahedron, 1984, 40, 2047.
[23] Cava, M.P.; Levinson, M.I. Tetrahedron,1985, 41, 5061*.*
[24] Varma, R.S.; Kumar, D. Org. Lett., 1999, 1, 697.
[25] Spychala, J. Tetrahedron, 2000, 56, 7981.
[26] Charette, A.B.; Grenon, M. J. Org. Chem., 2003, 68, 5792.
[27] Hurst, D.T.; Biggadike, K.; Tibble, J.J. Heterocycles, 1977, 6, 2005.
[28] Schlieper, C.A.; Wemple, J. Nucleosides & Nucleotides, 1984, 3, 369.
[29] Clark, J.; Pendergast, W. Journal of the Chemical Society [Section] C: Organic, 1969,2780.
[30] Daves, G.D., Jr.; O'Brien, D.E.; Lewis, L.R.; Cheng, C.C. J. Heterocycl. Chem., 1964, 1,130.
[31] Lipinski, C.A.; Craig, R.H.; Wright, R.B. J. Heterocycl. Chem., 1985, 22, 1723.
[32] Warczykowska, I; Wojciechowski, J. Polish Journal of Chemistry, 1980, 54, 335.
[33] Wagaw, S.; Buchwald, S.L. J. Org. Chem., 1996, 61, 7240.
[34] Wang, Q.M.; Fiol, C.J.; DePaoli-Roach, A.A.; Roach, P.J. J. Biol. Chem., 1994, 269, 14566.
[35] Bain, J.; McLauchlan, H.; Elliott, M.; Cohen, P. Biochem. J., 2003, 371, 199.
[36] Davies, S.P.; Reddy, H.; Caivano, M.; Cohen, P. Biochem. J., 2000, 351, 95.
[37] Korinek, V.; Barker, N.; Morin, P.J.; van Wichen, D.; de Weger, R.; Kinzler, K.W.; Vogelstein, B.; Clevers, H. Science, 1997, 275, 1784.
[38] Rubinfeld, B.; Robbins, P.; El-Gamil, M.; Albert, I.; Porfiri, E.; Polakis, P. Science, 1997, 275, 1790.
[39] Wang D, De la Fuente C, Deng L, Wang L, Zilberman I, Eadie C, Healey M, Stein D, Denny T, Harrison LE, Meijer L, Kashanchi F. Inhibition of human immunodeficiency virus type 1 transcription by chemical cyclin-dependent kinase inhibitors. J. Virol. 2001; 75: 7266-7279.
[40] Goedert, M. Curr. Opin. Gen. Dev., 2001, 11, 343.
[41] Sunkel et al., J. Cell Sci., 1988, 89, 25.
[42] Llamazares et al., Genes Dev., 1991, 5, 2153.
[43] Glover et al., Genes Dev., 1998, 12, 3777.
[44] Lee et al., Proc. Natl. Acad Sci. USA, 1998, 95, 9301.
[45] Leung et al., Nat. Struct. Biol, 2002, 9, 719.
[46] Kauselmann et al., EMBO J., 1999, 18, 5528.
[47] Nigg, Curr. Opin. Cell Biol., 1998, 10, 776.
[48] Yuan et al., Cancer Res., 2002, 62, 4186.
[49] Seong et al., J. Biol. Chem., 2002, 277, 32282.
[50] Lane et al., J Cell. Biol., 1996,135,1701.
[51] Cogswell et al., Cell Growth Differ., 2000,11, 615.
[52] Liu et al., Proc. Natl. Acad. Sci. USA, 2002, 99, 8672.
[53] Toyoshima-Morimoto et al., Nature, 2001, 410, 215.
[54] Roshak et al., Cell. Signalling, 2000,12, 405.
[55] Smits et al., Nat. Cell Biol., 2000, 2, 672.
[56] van Vugt et al., J Biol. Chem., 2001, 276, 41656.
[57] Sumara et al., Mol. Cell, 2002, 9, 515.
[58] Golan et al, J. Biol. Chem., 2002, 277, 15552.
[59] Kotani et al., Mol. Cell, 1998, 1, 371.
[60] Feng et al., Cell Growth Differ., 2001,12, 29.
[61] Dai et al., Oncogene, 2002, 21, 6195.
[62] Nurse, Nature, 1990, 344, 503.
[63] Nigg, Nat. Rev. Mol. Cell Biol., 2001, 2, 21.
[64] Hagting et al., EMBO J.; 1998,17,4127.
[65] Hagting et al., Curr. Biol., 1999, 9, 680.
[66] Yang et al., J Biol. Chem., 2001, 276, 3604.
[67] Takizawa et al., Curr. Opin. Cell Biol., 2000,12, 658.
[68] Seki et al., Mol. Biol. Cell, 1992, 3, 1373.
[69] Heald et al., Cell, 1993, 74, 463.
[70] Dalal et al., Mol. Cell. Biol., 1999,19, 4465.
[71] Toyoshima-Morimoto et al., Nature, 2001, 410, 215.
[72] Toyoshima-Morimoto et al., EMBO Rep., 2002, 3, 341.
[73] Wang et al., Mol. Cell. Biol., 2002, 22, 3450.

**Table 1. Example compounds**

| **No.** | **Structure** | **Name** |
|---|---|---|
| **II** | 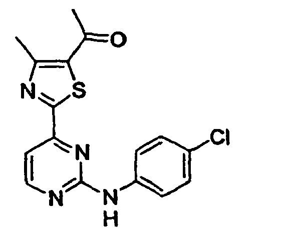 | 1-{2-[2-(4-Chloro-phenylamino)-pyrimidin-4-yl]-4-methyl-thiazol-5-yl}-ethanone |
| **III** | 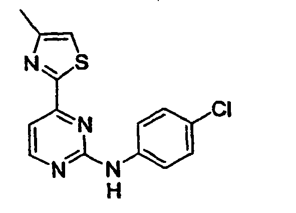 | (4-Chloro-phenyl)-[4-(4-methyl-thiazol-2-yl)-pyrimidin-2-yl]-amine |
| **IV** | 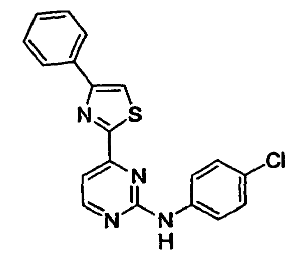 | (4-Chloro-phenyl)-[4-(4-phenyl-thiazol-2-yl)-pyrimidin-2-yl]-amine |
| **V** | 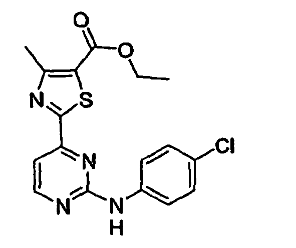 | 2-[2-(4-Chloro-phenylamino)-pyrimidin-4-yl]-4-methyl-thiazole-5-carboxylic acid ethyl ester |
| **VI** | 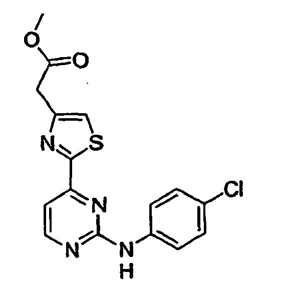 | {2-[2-(4-Chloro-phenylamino)-pyrimidin-4-yl]-thiazol-4-yl}-acetic acid methyl ester |
| **VII** | 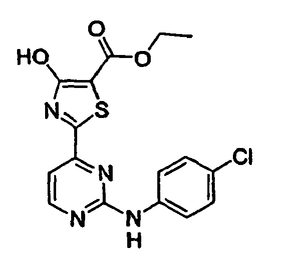 | 2-[2-(4-Chloro-phenylamino)-pyrimidin-4--yl]-4- hydroxy-thiazole-5-carboxylic acid ethyl ester |
| **VIII** | 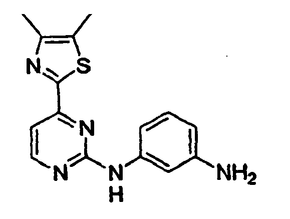 | N [4-(4,5-Dimethyl-thiazol-2-yl)-pyrinaidin-2- yl]-benzene-1,3-diamine |
| **IX** | 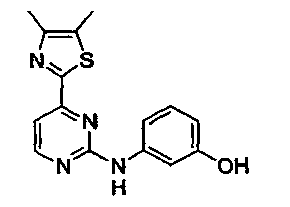 | 3-[4-(4,5-Dimethyl-thiazol-2-yl)-pyrimidin-2- ylamino]-phenol |
| **X** | 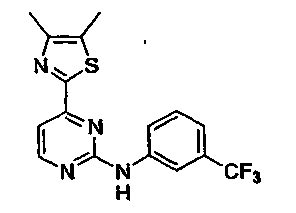 | [4-(4,5-Dimethyl-thiazol-2-yl)-pyrimidin-2-yl]- (3-trifluoromethyl-phenyl)-amine |
| **XI** | 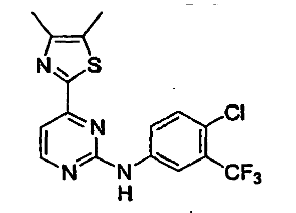 | (4-Chloro-3-trifluoromethyl-phenyl)-[4-(4,5- dimethyl-thiazol-2-yl)-pyrimidin-2-yl] -amine |
| **XII** | 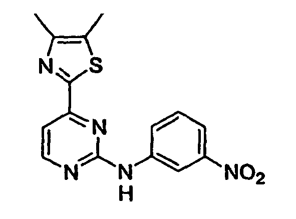 | [4-(4,5-Dimethyl-thiazol-2-yl)-pyrimidin-2-yl]- (3-nitro-phenyl)-amine |
| **XIII** | 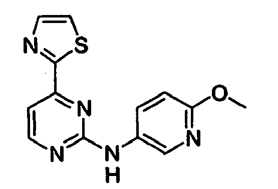 | (6-Methoxy-pyridin-3-yl)-[4-(4-thiazol-2-yl- pyrimidin-2-yl]-amine |
| **XIV** | 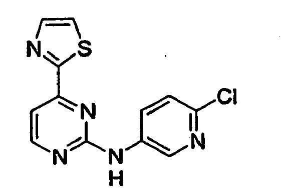 | (6-Chloro-pyridin-3-yl)-[4-(4-thiazol-2-yl- pyrimidin-2-yl]-amine |
| **XV** | 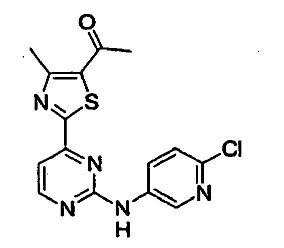 | 1-{2-[2-(4-Chloro-pyridin-3-ylamino)-pyrimidin-4-yl]-4-methyl-thiazol-5-yl}-ethanone |
| **XVI** | 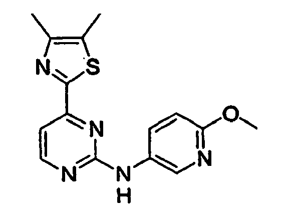 | [4-(4,5-Dimethyl-thiazol-2-yl)-pyrimidin-2-yl]- (3-methoxy-pyridin-3-yl)-amine |
| | 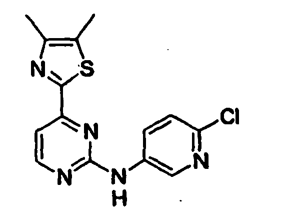 | (6-Chloro-pyridin-3-yl)-[4-(4,5-dimethyl-thiazol-2-yl)-pyrimidin-2-yl]-amine |
| **XVII XVIII** | 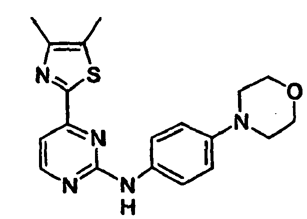 | [4-(4,5-Dimetbyl-thiazol-2-yl)-pyrimidin-2-yl]-(4-morpholin-4-yl-phenyl)-amine |
| **XIX** | 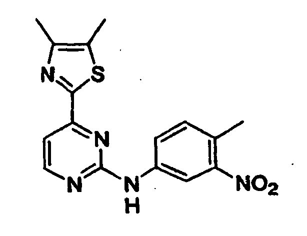 | [4-4,5-Dimethyl-thiazol-2-yl)-pyrimidin-2-yl]-(4-methyl-3-nitro-phenyl)-amine |
| **XX** | 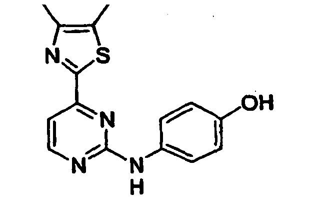 | 4-[4-(4,5-Dimethyl-thiazol-2-yl)-pyrimidin-2-ylamino]-phenol |
| **XXIII** | 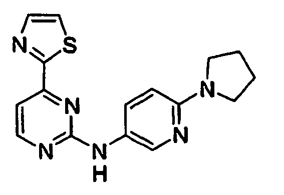 | (6-Pyrrolidin-1-yl-pyridin-3-yl)-(4-thiazol-2-yl-pyrimidin-2-yl)-amine |
| **XXIV** | 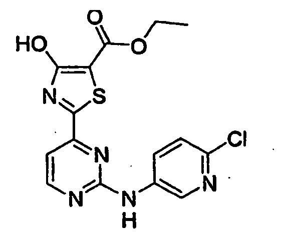 | 2-[2-(6-Chloro-pyridin-3-ylamino)-pyrimidin-4-yl]-4-hydroxy-thiazole-5-carboxylic acid ethyl ester |
| **XXV** | 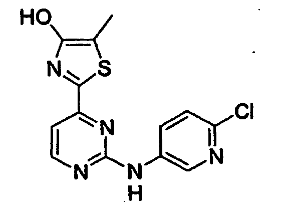 | 2-[2-(6-Chloro-pyridin-3-ylamino)-pyrimidin-4-yl]-5-methyl-thiazol-4-ol |
| **XXVI** | 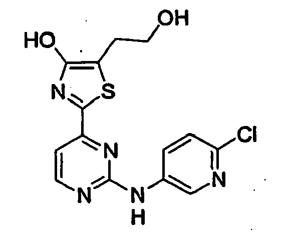 | 2-[2-(6-Chloro-pyridin-3-ylamino)-pyrimidin-4-yl]-5-(2-hydroxy-ethyl)-thiazol-4-ol |
| **XXVII** | 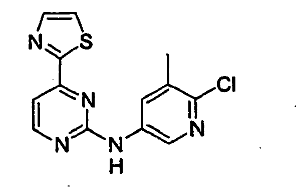 | (6-Chloro-5-methyl-pyridin-3-yl)-(4-thiazol-2-yl-pyrimidin-2-yl)-amine |

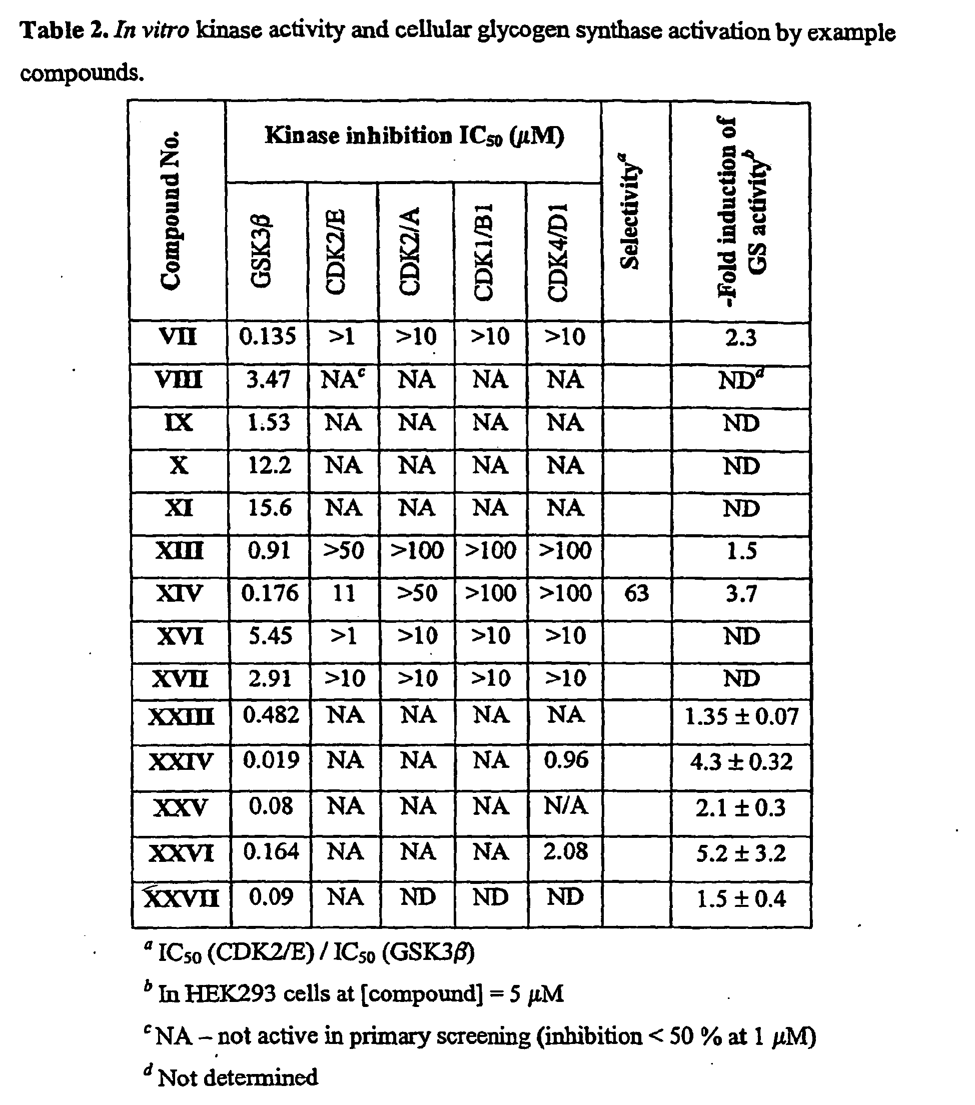

**Table 3. GSK versus CDK selectivity of example compound XIV.**

| **Enzyme** | ***K*ᵢ (µM)** | **Fold selectivity for GSK3** |
|---|---|---|
| GSK3 | 0.016 | - |
| CDK2/cyclin E | 5.5 | 345 |
| CDK1/cyclin B | > 100 | > 6,250 |
| CDK7/cyclin H | > 50 | > 3,125 |
| CDK4/cyclin D1 | > 100 | > 6,250 |
| CDK2/cyclin A | > 50 | > 3,125 |
| CDK9/cyclin T1 | 0.5 | 31 |

**Table 4. Activation of cellular glycogen synthase activity by example compound XIV.**

| **Test compound** | **Fold induction of GS activity** | |
|---|---|---|
| | HEK293 cells | 3T3 cells |
| 10 µM **XIV** | n/d | 4.8 |
| 5 µM **XIV** | 3.3 | 3.7 |
| 1 µM **XIV** | 1.6 | 2.5 |
| 0.5 µM **XIV** | 1.1 | n/d |
| 0.2 µM **XIV** | n/d | 1.1 |
| 0.1 µM **XIV** | 0.9 | n/d |
| Control | 1 | 1 |
| 40 mM LiCl | 3.7 | 2 |

**Table 5. Description of protein kinase panel.**

| | | **Screening** |
|---|---|---|
| **Kinase** | **Full name** | **[ATP]** |
| | | µm |
| AMPK | AMP-activated protein kinase | 50 |
| CDK2-cyclin A | Cyclic dependent kinase 2 - cyclin A complex | 20 |
| CHK1 | Checkpoint kinase-1 | 20 |
| CK1 | Casein kinase-1 | 20 |
| CK2 | Casein kinase-2 | 5 |
| CSK | C-terminal Src kinase | 20 |
| DYRK1A | Dual specificity tyrosine phosphorylation regulated kinase 1A | 50 |
| GSK3 - beta | Glycogen synthase kinases 3-beta | 5 |
| JNK alpha 1 | c-Jun terminal kinase | 20 |
| LCK | Lymphocyte kinase | 50 |
| MAPK2/ERK2 | Mitogen activated protein kinase | 50 |
| MAPKAP-K1a | MAPK - activated protein kinase -1a | 50 |
| MAPKAP - K2 | MAPK - activated protein kinase - 2 | 20 |
| MKK1 | MAPK kinase | 5 |
| MSK1 | Mitogen and stress activated protein kinase - 1 | 20 |
| P70S6K | P70 ribosomal protein S6 kinase | 20 |
| PDK1 | 3-phosphoinositide-dependent protein kinase -1 | 20 |
| PHK | Phosphorylase kinase | 20 |
| PKA | Cyclin AMP dependent protein kinase | 5 |
| PKB-alpha | Protein kinase B | 5 |
| PKC-alpha | Protein kinase C | 20 |
| PRANK | P38 regulated/activated kinase | 20 |
| ROCK-II | Rho-dependent protein kinase | 20 |
| SAPK2a/p38 | Stress activated protein kinase - 2a | 50 |
| SAPK2b/p38-beta2 | Stress activated protein kinase - 2b | 20 |
| SAPK3/p38-gamma | Stress activated protein kinase - 3 | 5 |
| SAPK4/p38-delta | Stress activated protein kinase - 4 | 5 |
| SGK | Serum and glucocorticoid activated kinase | 20 |
| NEK6 | NIMA family kinase 6 | 50 |

**Table 6. Kinase selectivity screen of example compound XIV (1 µM).**

| **Enzyme** | **% Activity** |
|---|---|
| MKK1 | 83±4 |
| MAPK2/ERK2 | 84 ± 8 |
| JNK/SAPK1c | 56 ± 4 |
| SAPK2a/p38 | 84 ± 7 |
| SAPK2b/p38b2 | 92 ± 4 |
| SAPK3/p38g | 88 ± 3 |
| SAPK4/p38d | 91 ± 9 |
| MAPKAP-K1a | 60 ± 1 |
| MAPKAP-K2 | 86 ± 7 |
| MSK1 | 90 ± 6 |
| PRAK | 83±3 |
| PKA | 76 ± 3 |
| PKCa | 76 ± 0 |
| PDK1 | 85 ± 5 |
| PKBdelta PH | 98 ± 1 |
| SGK | 79 ± 4 |
| p70S6K | 91 ± 2 |
| GSK3b | 13 ± 5 |
| ROCK-II | 87 ± 6 |
| AMPK | 82 ± 5 |
| CDK1 | 78 ± 4 |
| CK2 | 84 ± 1 |
| PHK | 120 ± 7 |
| Lck | 66 ± 5 |
| CSK | 93 ± 8 |
| CK1 | 69 ± 1 |
| DYRK1 a | 74 ± 8 |
| NEK6 | 87 ± 6 |

## Claims

1. A compound of formula I, or a pharmaceutically acceptable salt thereof, 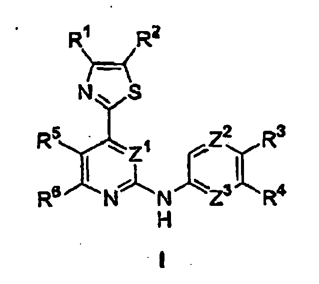 wherein:
Z¹ is N;
Z² is N;
Z³ is CR⁷;
R¹, R², R³, R⁴, R⁵, R⁶, and R⁷ are each independently H, R⁸, or R⁹;
each R⁸ is independently a C₁₋₆-alkyl group, a C₆₋₁₀-aryl group or a cycloheteroalkyl group, said cycloheteroalkyl group being a cyclic heteroalkyl group, wherein said heteroalkyl group is a C₁₋₆-alkyl group comprising one or more heteroatoms;
each R⁹ is independently halo, NO₂, C₁₋₆-alkoxy, CN, CF₃, SO₃H, SO₂NR¹⁰R¹¹, SO₂R¹², NR¹³R¹⁴, (CH₂)ₐCOOR¹⁵, (CH₂)_{b}CONR¹⁶R¹⁷, (CH₂)_{c}COR¹⁸ or (CH₂)_{d}OH;
a, b, c and d are each independently 0, 1 2 3 or 4;
R¹⁰⁻¹⁸ are each independently H or C₁₋₆-alkyl.

2. A compound according to claim 1 wherein each R⁹ is independently halo, NO₂, C₁₋₆₋alkoxy, CN, CF₃, SO₃H, SO₂NH₂, SO₂Me, OH, NH₂, (CH₂)ₐCOOR¹⁵, (CH₂)_{d}OH, CONH₂ or COR¹⁸.

3. A compound according to any preceding claim wherein:
R¹ is H, C₁₋₆-alkyl, C₆₋₁₀-aryl, (CH₂)ₐCOOR¹⁵ or OH;
R² is H, (CH₂)_{d}OH, (CH₂)ₐCOOR¹⁵ , COR¹⁸ or C₁₋₆-alkyl;
R³ is halo, H, C₁₋₆-alkoxy, a cycloheteroalkyl group as defined in claim 1, C₁₋₆-alkyl or OH;
R⁴ is H, NH₂, OH, C₁₋₆-alkyl, CF₃ or NO₂; and
R⁵ and R⁶ are both H.

4. A compound according to any preceding claim wherein:
R¹ is H, Me, Ph, CH₂COOMe or OH;
R² is H, (CH₂)₂OH, COOEt, COMe or Me;
R³ is Cl, H, OMe, N-morpholinyl, N-pyrrolidinyl, Me or OH;
R⁴ is H, NH₂, OH, Me, CF₃ or NO₂; and
R⁵ and R⁶ are both H.

5. A compound according to claim 1 wherein:
R¹ is H, OH or C₁₋₆-alkyl.
R² is H, (CH₂)_{d}OH, C₁₋₆-alkyl, (CH₂)ₐCOOR¹⁵, COR¹⁸;
R³ is halo, C₁₋₆-alkoxy or a heterocycloalkyl group as defined in claim 1;
R⁴ is H or C₁₋₆-alkyl; and
Z³ is CH.

6. A compound according to claim 5 wherein:
R¹ is H, OH or Me;
R² is H, (CH₂)₂OH, Me, COOEt, COMe;
R³ is halo, OMe or N-pyrrolidinyl;
R⁴ is H or Me; and
Z³ is CH.

7. A compound according to claim 1 which is selected from the following:
(6-Methoxy-pyridin-3-yl)-(4-thiazol-2-yl-pyrimidin-2-yl)-amine
(6-Chloro-pyridin-3-yl)-(4-thiazol-2ryl-pyrimidin-2-yl)-amine
1-{2-[2-(6-Chloro-pyridin-3-ylamino)-pyrimidin-4-yl]-4-methyl-thiazol-5-yl)-ethanone
[4-(4,5-Dimethyl-thiazol-2-yl)-pyrimidin-2-yl]-(6-methoxy-pyridin-3-yl)-amine
(6-Chloro-pyridin-3-yl)-[4-(4,5-dimethyl-thiazol-2-yl)-pyrimidin-2-yl]-amine
(6-Pyrrolidin-1-yl-pyridin-3-yl)-(4-thiazol-2-yl-pyrimidin-2-yl)-amine
2-[2-(6-Chloro-pyridin-3-ylamino)-pyrimidin-4-yl]-4-hydroxy-thiazole-5-carboxylic acid ethyl ester
2-[2-(6-Chloro-pyridin-3-ylamino)-pyriraidin-4-yl]-5-methyl-thiazol-4-ol
2-[2-(6-Chloro-pyridin-3-ylamino)-pyrimidin-4-yl]-5-(2-hydroxy-ethyl)-thiazol-4-ol
(6-Chloro-5-methyl-pyridin-3-yl)-(4-thiazol-2-yl-pyrimidin-2-yl)-amine.

8. A compound according to claim 1 which is selected from the following:
(6-Methoxy-pyridin-3-yl)-(4-thiazol-2-yl-pyrimidin-2-yl)-amine
(6-Chloro-pyridin-3-yl)-(4-thiazol-2-yl-pyrimidin-2-yl)-amine
[4-(4,5-Dimethyl-thiazol-2-yl)-pyramidin-2-yl]-(6-methoxy-pyridin-3-yl)-amine
(6-Pyrrolidin-1-yl-pyadin-3-yl)-(4-thiazol-2-yl-pyrimidin-2-yl)-amine
2-[2-(6-Chloro-pyridin-3-ylamino)-pyrimidin-4-yl]-4-hydroxy-thiazole-5-carboxylic acid ethyl ester
2-[2-(6-Chloro-pyridin-3-ylamino)-pyrimidin-4-yl]-5-methyl-thiazol-4-ol
2-[2-(6-Chloro-pyridin-3-ylamino)-pyrimidin-4-yl]-5-(2-hydroxy-ethyl)-thiazol-4-ol
(6-Chloro-5-methyl-pyridin-3-yl)-(4-thiazol-2-yl-pyrimidin-2-yl)-amine.

9. A compound according to claim 1 which is selected from the following:
(6-Methoxy-pyridin-3-yl)-(4-thiazol-2-yl-pyrimidin-2-yl)-amine; and
(6-Chloro-pyridin-3-yl)-(4-thiazol-2-yl-pyrimidin-2-yl)-amine
(6-Pyrrolidin-1-yl-pyridin-3-yl)-(4-thiazol-2-yl-pyrimidin-2-yl)-amine
2-[2-(6-Chloro-pyridin-3-ylamino)-pyrimidin-4-yl]-4-hydroxy-thiazole-5-cxboxylic acid ethyl ester
2-[2-(6-Chloro-pyridin-3-ylamino)-pyrimidin-4-yl]-5-methyl-thiazol-4-ol
2-[2-(6-Chloro-pyridin-3-ylamino)-pyrimidin-4-yl]-5-(2-hydroxy ethyl)-thiazol-4-ol
(6-Chloro-5-methyl-pyridin-3-yl)-(4-thiazol-2-yl-pyrimidin-2-yl)-amine

10. A compound according to claim 1 which is (6-Chloro-pyridin-3-yl)-(4-thiazol-2-yl-pyrimidin-2-yl)-amine.

11. A compound of formula Ia, or a pharmaceutically acceptable salt thereof, 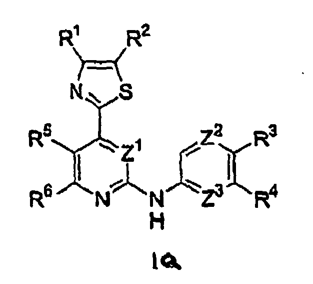 wherein:
Z¹ is N;
R¹ is alkyl, aryl, OH or (CH₂)ₐCOOR¹⁵;
R² is COR¹⁸, H, COOR¹⁵ or alkyl;
R³ is halo, H, OH, alkyl or morpholino;
R⁴ is H, NH₂, OH, CF₃ or NO₂; and
Z² and Z³ are both CH;
a is 0, 1 2 3 or 4;
R¹⁵ and R¹⁸ are each independently H or C₁₋₆-alkyl.

12. A compound according to claim 11 wherein:
R¹ is Me, Ph, OH or CH₂COOMe;
R² is COMe, H, COOEt or Me; and
R³ is halo, H, OH, C₁₋₆-alkyl or morpholino.

13. A compound according to claim 11 which is selected from the following:
1-{2-(2-(4-Chloro-phenylamino)-pyrimidin-4-yl]-4-methyl-thiazol-5-yl}-ethanone
(4-Chloro-phenyl)-[4-(4-methyl-thiazol-2-yl)-pyriznidin-2-yl]-amine
(4-Chloro-phenyl)-[4-(4-phenyl-thiazol-2-yl)-pyrimidin-2-yl]-amine
2-[2-(4-Chloro-phenylamino)-pyrimidin-4-yl]-4-methyl-thiazole-5-carboxylic acid ethyl ester
(2-[2-(4-Chloro-phenylamino)-pyrimidin-4-yl]-thiazol-4-yl)-acetic acid methyl ester 2-[2-(4-Chloro-phentylamino)-pyrimidin-4-yl]-4-hydroxy-thiazole-5-carboxylic acid ethyl ester
*N*-[4-(4,5-Dimethyl-thiazol-2-yl)-pyrimidin-2-yl]-benzene-1,3-diamine
3-[4-(4,5-Dimethyl-thiazol-2-yl)-pyrimidin-2-ylamino]-phenol
[4-(4,5-Dimethyl-thiazol-2-yl)-pyrimidin-2-yl]-(3-trifluoromethyl-phenyl)-amine (4-Chloro-3-trifluoromethyl-phenyl)-[4-(4,5-dimethyl-thiazol-2-yl)-pyrimidin-2-yl]-amine
[4-(4,5-Dimethyl-thiazol-2-yl)-pyrimidin-2-yl]-(3-nitro-phenyl)-amine
[4-(4,5-Dimethyl-thiazol-2-yl)-pyrimidin-2-yl]-(4-morpholin-4-yl-phenyl)-amine
[4-(4,5-Dimethyl-thiazol-2-yl)-pyrimidin-2-yl]-(4-methyl-3-nitro-phenyl)-amine
4-[4-(4,5-Dimethyl-thiazol-2-yl)-pyrimidin-2-ylamino]-phenol.

14. A pharmaceutical composition comprising a compound according to any preceding claim admixed with a pharmaceutically acceptable diluent, excipient or carrier.

15. Use of a compound according to any one of claims 1 to 13 in the preparation of a medicament for treating a proliferative disorder.

16. Use according to claim 15 wherein the proliferative disorder is cancer or leukemia.

17. Use according to claim 15 wherein the proliferative disorder is glomerulonephritis, rheumatoid arthritis, psoriasis or chronic obstructive pulmonary disorder.

18. Use of a compound according to any one of claims 1 to 13 in the preparation of a medicament for treating a viral disorder.

19. Use according to claim 18 wherein the viral disorder is selected from human cytomegalovirus (HCMV), herpes simplex virus type 1 (HSV-1), human immunodeficiency virus type 1 (HSV-1), and varicella zoster virus (VZV).

20. Use of a compound according to any one of claims 1 to 13 in the preparation of a medicament for treating a CNS disorder.

21. Use according to claim 20 wherein the CNS disorder is Alzheimer's disease or bipolar disorder.

22. Use of a compound according to any one of claims 1 to 13 in the preparation of a medicament for treating alopecia.

23. Use of a compound according to any one of claims 1 to 13 in the preparation of a medicament for treating a stroke.

24. Use of a compound according to any one of claims 1 to 13 in the preparation of a medicament for treating diabetes.

25. Use according to claim 24 wherein the diabetes is non-insulin-dependent diabetes or Type II diabetes.

26. Use of a compound according to any one of claims 1 to 13 in the preparation of a medicament for treating an inflammatory diseases or an infectious disease.

27. Use of a compound according to any one of claims 1 to 13 in an assay for identifying further candidate compounds capable of inhibiting one or more of a cyclin dependent kinase, aurora kinase, GSK and a PLK enzyme.

28. Use according to claim 27 wherein said assay is a competitive binding assay.

29. A process for preparing a compound of formula I as defined in claim 1 or a compound of formula Ia as defined in claim 11, said process comprising reacting a compound of formula 9 with a compound of formula 10 to form a compound of formula I, wherein R¹⁻⁶ are as defined previously.

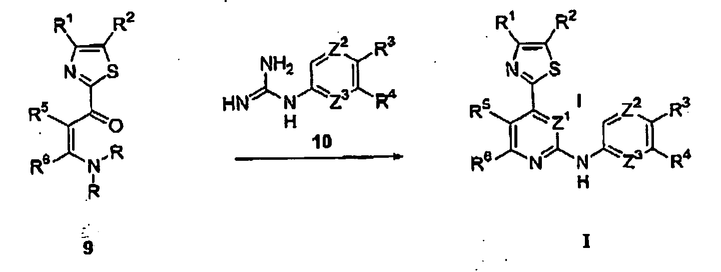

30. A process for preparing a compound of formula I as defined in claim 1 or a compound of formula **Ia** as defined in claim 11, said process comprising reacting a compound of formula 15 with a compound of formula 3 to form a compound of formula I, wherein R¹⁻⁶ are as defined previously.

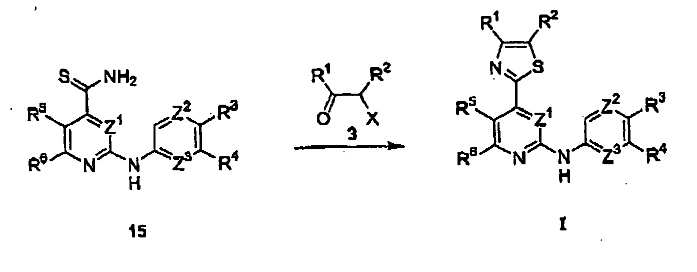

## Patentansprüche

1. Verbindung der Formel I oder pharmazeutisch verträgliches Salz davon, 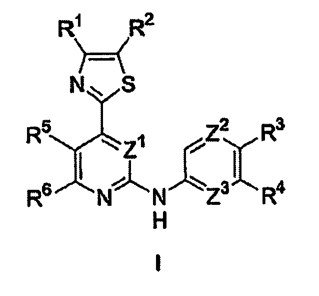 wobei:
Z¹ N ist,
Z² N ist,
Z³ CR⁷ ist,
R¹, R², R³, R⁴, R⁵, R⁶ und R⁷ jeweils unabhängig voneinander H, R⁸ oder R⁹ sind,
jeder R⁸ unabhängig voneinander eine C₁₋₆-Alkylgruppe, eine C₆-₁₀-Arylgruppe oder eine Cycloheteroalkylgruppe ist, wobei die Cycloheteroalkylgruppe eine zyklische Heteroalkylgruppe ist, wobei die Heteroalkylgruppe eine C₁₋₆-Alkylgruppe ist, die ein oder mehrere Heteroatome umfaßt, jeder R⁹ unabhängig voneinander Halogen, NO₂, C₁₋₆-Alkoxy, CN, CF₃, SO₃H, SO₂NR¹⁰R¹¹, SO₂R¹², NR¹³R¹⁴, (CH₂)ₐCOOR¹⁵, (CH₂)_{b}CONR¹⁶R¹⁷, (CH₂)_{c}COR¹⁸ oder (CH₂)_{d}OH ist,
a, b, c und d jeweils unabhängig voneinander 0, 1, 2, 3 oder 4 sind,
R¹⁰⁻¹⁸ jeweils unabhängig voneinander H oder C₁₋₆-Alkyl sind.

2. Verbindung nach Anspruch 1, wobei jeder R⁹ unabhängig voneinander Halogen, NO₂, C₁₋₆-Alkoxy, CN, CF₃, SO₃H, SO₂NH₂, SO₂Me, OH, NH₂, (CH₂)ₐCOOR¹⁵, (CH₂)_{d}OH, CONH₂ oder COR¹⁸ ist.

3. Verbindung nach einem der vorangegangenen Ansprüche, wobei:
R¹ H, C₁₋₆-Alkyl, C₆₋₁₀-Aryl, (CH₂)ₐCOOR¹⁵ oder OH ist,
R² H, (CH₂)_{d}OH, (CH₂)ₐCOOR¹⁵, COR¹⁸ oder C₁₋₆-Alkyl ist,
R³ Halogen, H, C₁₋₆-Alkoxy, eine Cycloheteroalkylgruppe wie in Anspruch 1 definiert, C₁₋₆-Alkyl oder OH ist,
R⁴ H, NH₂, OH, C₁₋₆-Alkyl, CF₃ oder NO₂ ist und
R⁵ und R⁶ beide H sind.

4. Verbindung nach einem der vorangegangenen Ansprüche, wobei:
R¹ H, Me, Ph, CH₂COOMe oder OH ist,
R² H, (CH₂)₂OH, COOEt, COMe oder Me ist,
R³ Cl, H, OMe, N-Morpholinyl, N-Pyrrolidinyl, Me oder OH ist,
R⁴ H, NH₂, OH, Me, CF₃ oder NO₂ ist und
R⁵ und R⁶ beide H sind.

5. Verbindung nach Anspruch 1, wobei:
R¹ H, OH oder C₁₋₆-Alkyl ist,
R² H, (CH₂)_{d}OH, C₁₋₆-Alkyl, (CH₂)ₐCOOR¹⁵ oder COR¹⁸ ist,
R³ Halogen, C₁₋₆-Alkoxy oder eine wie in Anspruch 1 definierte Heterocycloalkylgruppe ist, R⁴ H oder C₁₋₆-Alkyl ist und
Z³ CH ist.

6. Verbindung nach Anspruch 5, wobei:
R¹ H, OH oder Me ist,
R² H, (CH₂)₂OH, Me, COOEt, COMe ist,
R³ Halogen, OMe oder N-Pyrrolidinyl ist,
R⁴ H oder Me ist und
Z³ CH ist.

7. Verbindung nach Anspruch 1, welche unter den folgenden ausgewählt ist:
(6-Methoxy-pyridin-3-yl)-(4-thiazol-2-yl-pyrimidin-2-yl)-amin,
(6-Chlor-pyridin-3-yl)-(4-thiazol-2-yl-pyrimidin-2-yl)-amin,
1-{2-[2-(6-Chlor-pyridin-3-ylamino)-pyrimidin-4-yl]-4-methyl-thiazol-5-yl}-ethanon,
[4-(4,5-Dimethyl-thiazol-2-yl)-pyrimidin-2-yl]-(6-methoxy-pyridin-3-yl)-amin,
(6-Chlor-pyridin-3-yl)-[4-(4,5-dimethyl-thiazol-2-yl)-pyrimidin-2-yl]-amin,
(6-Pyrrolidin-1-yl-pyridin-3-yl)-(4-thiazol-2-yl-pyrimidin-2-yl)-amin,
2-[2-(6-Chlor-pyridin-3-ylamino)-pyrimidin-4-yl]-4-hydroxy-thiazol-5-carbonsäureethylester,
2-[2-(6-Chlor-pyridin-3-ylamino)-pyrimidin-4-yl]-5-methyl-thiazol-4-ol,
2-[2-(6-Chlor-pyridin-3-ylamino)-pyrimidin-4-yl]-5-(2-hydroxy-ethyl)-thiazol-4-ol,
(6-Chlor-5-methyl-pyridin-3-yl)-(4-thiazol-2-yl-pyrimidin-2-yl)-amin.

8. Verbindung nach Anspruch 1, welche unter den folgenden ausgewählt ist:
(6-Methoxy-pyridin-3-yl)-(4-thiazol-2-yl-pyrimidin-2-yl)-amin,
(6-Chlor-pyridin-3-yl)-(4-thiazol-2-yl-pyrimidin-2-yl)-amin,
[4-(4,5-Dimethyl-thiazol-2-yl)-pyrimidin-2-yl]-(6-methoxy-pyridin-3-yl)-amin,
(6-Pyrrolidin-1-yl-pyridin-3-yl)-(4-thiazol-2-yl-pyrimidin-2-yl)-amin,
2-[2-(6-Chlor-pyridin-3-ylamino)-pyrimidin-4-yl]-4-hydroxy-thiazol-5-carbonsäureethylester,
2-[2-(6-Chlor-pyridin-3-ylamino)-pyrimidin-4-yl]-5-methyl-thiazol-4-ol,
2-[2-(6-Chlor-pyridin-3-ylamino)-pyrimidin-4-yl]-5-(2-hydroxy-ethyl)-thiazol-4-ol,
(6-Chlor-5-methyl-pyridin-3-yl)-(4-thiazol-2-yl-pyrimidin-2-yl)-amin.

9. Verbindung nach Anspruch 1, welche unter den folgenden ausgewählt ist:
(6-Methoxy-pyridin-3-yl)-(4-thiazol-2-yl-pyrimidin-2-yl)-amin und
(6-Chlor-pyridin-3-yl)-(4-thiazol-2-yl-pyrimidin-2-yl)-amin,
(6-Pyrrolidin-1-yl-pyridin-3-yl)-(4-thiazol-2-yl-pyrimidin-2-yl)-ami n,
2-[2-(6-Chlor-pyridin-3-ylamino)-pyrimidin-4-yl]-4-hydroxy-thiazol-5-carbonsäureethylester,
2-[2-(6-Chlor-pyridin-3-ylamino)-pyrimidin-4-yl]-5-methyl-thiazol-4-ol,
2-[2-(6-Chlor-pyridin-3-ylamino)-pyrimidin-4-yl]-5-(2-hydroxy-ethyl)-thiazol-4-ol,
(6-Chlor-5-methyl-pyridin-3-yl)-(4-thiazol-2-yl-pyrimidin-2-yl)-amin.

10. Verbindung nach Anspruch 1, welche (6-Chlor-pyridin-3-yl)-(4-thiazol-2-yl-pyrimidin-2-yl)-amin ist.

11. Verbindung der Formel la oder ein pharmazeutisch verträgliches Salz davon, 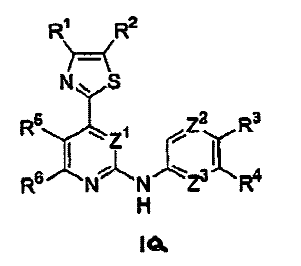 wobei:
Z¹ N ist,
R¹ Alkyl, Aryl, OH oder (CH₂)ₐCOOR¹⁵ ist,
R² COR¹⁸, H, COOR¹⁵ oder Alkyl ist,
R³ Halogen, H, OH, Alkyl oder Morpholino ist,
R⁴ H, NH₂, OH, CF₃ oder NO₂ ist und
Z² und Z³ beide CH sind,
a 0, 1, 2, 3 oder 4 ist,
R¹⁵ und R¹⁸ jeweils unabhängig voneinander H oder C₁₋₆-Alkyl sind.

12. Verbindung nach Anspruch 11, wobei:
R¹ Me, Ph, OH oder CH₂COOMe ist,
R² COMe, H, COOEt oder Me ist und
R³ Halogen, H, OH, C₁₋₆-Alkyl oder Morpholino ist.

13. Verbindung nach Anspruch 11, welche unter den folgenden ausgewählt ist:
1-{2-(2-(4-Chlor-phenylamino)-pyrimidin-4-yl]-4-methyl-thiazol-5-yl}-ethanon,
(4-Chlor-phenyl)-[4-(4-methyl-thiazol-2-yl)-pyrimidin-2-yl]-amin,
(4-Chlor-phenyl)-[4-(4-phenyl-thiazol-2-yl)-pyrimidin-2-yl]-amin,
2-[2-(4-Chlor-phenylamino)-pyrimidin-4-yl]-4-methyl-thiazol-5-carbonsäureethylester,
{2-[2-(4-Chlor-phenylamino)-pyrimidin-4-yl]-thiazol-4-yl]-essigsäuremethylester,
2-[2-(4-Chlor-phenylamino)-pyrimidin-4-yl]-4-hydroxy-thiazol-5-carbonsäureethylester,
N-[4-(4,5-Dimethyl-thiazol-2-yl)-pyrimidin-2-yl]-benzen-1,3-diamin,
3-[4-(4,5-Dimethyl-thiazol-2-yl)-pyrimidin-2-ylamino]-phenol,
[4-(4,5-Dimethyl-thiazol-2-yl)-pyrimidin-2-yl]-(3-trifluormethyl-phenyl)-amin,
(4-Chlor-3-trifluormethyl-phenyl)-[4-(4,5-dimethyl-thiazol-2-yl)-pyrimidin-2-yl]-amin,
[4-(4,5-Dimethyl-thiazol-2-yl)-pyrimidin-2-yl]-(3-nitro-phenyl)-amin,
[4-(4,5-Dimethyl-thiazol-2-yl)-pyrimidin-2-yl]-(4-morpholin-4-yl-phenyl)-amin,
[4-(4,5-Dimethyl-thiazol-2-yl)-pyrimidin-2-yl]-(4-methyl-3-nitro-phenyl)-amin,
4-[4-(4,5-Dimethyl-thiazol-2-yl)-pyrimidin-2-ylamino]-phenol.

14. Pharmazeutische Zusammensetzung, welche eine Verbindung nach einem der vorangegangenen Ansprüche im Gemisch mit einem pharmazeutisch verträglichen Verdünnungsmittel, Hilfsstoff oder Träger umfaßt.

15. Verwendung einer Verbindung nach einem der Ansprüche 1 bis 13 bei der Herstellung eines Medikaments zur Behandlung einer proliferativen Störung.

16. Verwendung nach Anspruch 15, wobei die proliferative Störung Krebs oder Leukämie ist.

17. Verwendung nach Anspruch 15, wobei die proliferative Störung Glomerulonephritis, rheumatoide Arthritis, Psoriasis oder chronisch-obstruktive Lungenstörung ist.

18. Verwendung einer Verbindung nach einem der Ansprüche 1 bis 13 bei der Herstellung eines Medikaments zur Behandlung einer viralen Störung.

19. Verwendung nach Anspruch 18, wobei die virale Störung unter humanem Cytomegalovirus (HCMV), Herpes simplex-Virus Typ 1 (HSV-1), humanem Immunschwächevirus Typ 1 (HIV-1) und Varicella zoster-Virus (VZV) ausgewählt ist.

20. Verwendung einer Verbindung nach einem der Ansprüche 1 bis 13 bei der Herstellung eines Medikaments zur Behandlung einer ZNS-Störung.

21. Verwendung nach Anspruch 20, wobei die ZNS-Störung Alzheimer'sche Krankheit oder bipolare Störung ist.

22. Verwendung einer Verbindung nach einem der Ansprüche 1 bis 13 bei der Herstellung eines Medikaments zur Behandlung von Alopezie.

23. Verwendung einer Verbindung nach einem der Ansprüche 1 bis 13 bei der Herstellung eines Medikaments zur Behandlung eines Schlaganfalls.

24. Verwendung einer Verbindung nach einem der Ansprüche 1 bis 13 bei der Herstellung eines Medikaments zur Behandlung von Diabetes.

25. Verwendung nach Anspruch 24, wobei der Diabetes nicht-insulinpflichtiger Diabetes oder Typ II-Diabetes ist.

26. Verwendung einer Verbindung nach einem der Ansprüche 1 bis 13 bei der Herstellung eines Medikaments zur Behandlung einer inflammatorischen Störung oder einer infektiösen Störung.

27. Verwendung einer Verbindung nach einem der Ansprüche 1 bis 13 in einem Test zum Identifizieren weiterer Kandidatenverbindungen, die eine(s) oder mehrere unter einer cyclinabhängigen Kinase, Aurorakinase, GSK und einem PLK-Enzym hemmen können.

28. Verwendung nach Anspruch 27, wobei der Test ein kompetitiver Bindungstest ist.

29. Verfahren zum Herstellen einer Verbindung der Formel I, wie in Anspruch 1 definiert, oder einer Verbindung der Formel Ia, wie in Anspruch 11 definiert, wobei das Verfahren das Umsetzen einer Verbindung der Formel 9 mit einer Verbindung der Formel 10 unter Bildung einer Verbindung der Formel I umfaßt, wobei R¹⁻⁶ wie zuvor definiert sind.

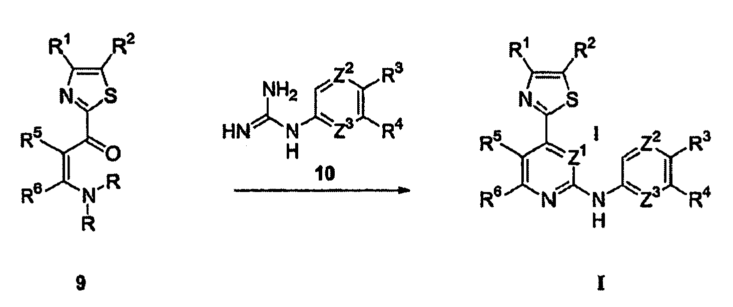

30. Verfahren zum Herstellen einer Verbindung der Formel I, wie in Anspruch 1 definiert, oder einer Verbindung der Formel Ia, wie in Anspruch 11 definiert, wobei das Verfahren das Umsetzen einer Verbindung der Formel 15 mit einer Verbindung der Formel 3 unter Bildung einer Verbindung der Formel I umfaßt, wobei R¹⁻⁶ wie zuvor definiert sind.

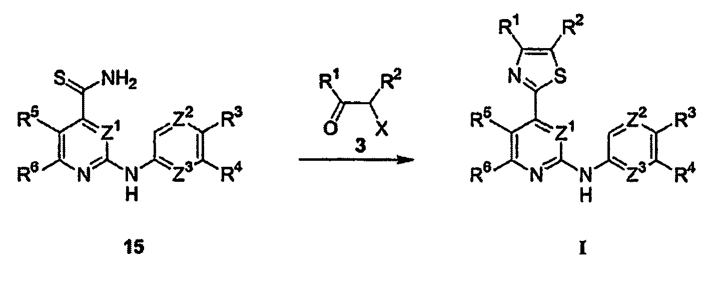

## Revendications

1. Composé de formule I, ou sel pharmaceutiquement acceptable de celui-ci, 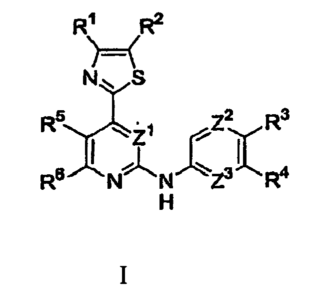 où :
Z¹ est N ;
Z² est N ;
Z³ est CR⁷ ;
R¹, R², R³, R⁴, R⁵, R⁶ et R⁷ sont chacun indépendamment H, R⁸ ou R⁹ ;
chaque R¹ est indépendamment un groupe C₁-₆-alkyle, un groupe C₆₋₁₀-aryle ou un groupe cyclohétéroalkyle, ledit groupe cyclohétéroalkyle étant un groupe hétéroalkyle cyclique, où ledit groupe hétéroalkyle est un groupe C₁₋₆-alkyle comprenant un ou plusieurs hétéroatomes ;
chaque R⁹ est indépendamment halo, NO₂, C₁₋₆-alcoxy, CN, CF₃, SO₃H, SO₂NR¹⁰R¹¹, SO₂R¹², NR¹³R¹⁴, (CH₂)ₐCOOR¹⁵, (CH₂)_{b}CONR¹⁶R¹⁷, (CH₂)_{c}COR¹⁸ ou (CH₂)_{d}OH ;
a, b, c et d sont chacun indépendamment 0, 1, 2, 3 ou 4 ;
les R¹⁰⁻¹⁸ sont chacun indépendamment H ou C₁₋₆-alkyle.

2. Composé selon la revendication 1 où chaque R⁹ est indépendamment halo, NO₂, C₁₋₆-alcoxy, CN, CF₃, SO₃H, SO₂NH₂, SO₂Me, OH, NH₂, (CH₂)ₐCOOR¹⁵, (CH₂)_{d}OH, CONH₂ ou COR¹⁸.

3. Composé selon l'une quelconque des revendications précédentes où:
R¹ est H, C₁₋₆-alkyle, C₆₋₁₀-aryle, (CH₂)ₐCOOR¹⁵ ou OH ;
R² est H, (CH₂)_{d}OH, (CH₂)ₐCOOR¹⁵, COR¹⁸ ou C₁₋₆-alkyle ;
R³ est halo, H, C₁₋₆-alcoxy, un groupe hétérocycloalkyle tel que défini dans la revendication 1, C₁₋₆-alkyle ou OH ;
R⁴ est H, NH₂, OH, C₁₋₆-alkyle, CF₃ ou NO₂ ; et
R⁵ et R⁶ sont l'un et l'autre H.

4. Composé selon l'une quelconque des revendications précédentes où :
R¹ est H, Me, Ph, CH₂COOMe ou OH ;
R² est H, (CH₂)₂OH, COOEt, COMe ou Me ;
R³ est Cl, H, OMe, N-morpholinyle, N-pyrrrolidinyle, Me ou OH ;
R⁴ est H, NH₂, OH, Me, CF₃ ou NO₂ ; et
R⁵ et R⁶ sont l'un et l'autre H.

5. Composé selon la revendication 1 où :
R¹ est H, OH ou C₁₋₆-alkyle ;
R² est H, (CH₂)_{d}OH, C₁₋₆-alkyle, (CH₂)ₐCOOR¹⁵, COR¹⁸ ;
R³ est halo, H, C₁₋₆-alcoxy ou un groupe hétérocycloalkyle tel que défini dans la revendication 1 ;
R⁴ est H ou C₁₋₆-alkyle; et
Z³ est CH.

6. Composé selon la revendication 5 où :
R¹ est H, OH ou Me ;
R² est H, (CH₂)₂OH, Me, COOEt, COMe ;
R³ est halo, H, OMe ou N-pyrrolidinyle ;
R⁴ est H ou Me ; et
Z³ est CH.

7. Composé selon la revendication 1 qui est choisi parmi les suivants :
(6-méthoxy-pyridin-3-yl)-(4-thiazol-2-yl-pyrimidin-2-yl)-amine
(6-chloro-pyridin-3-yl)-(4-thiazol-2-yl-pyrimidin-2-yl)-amine
1-{2-[2-(6-chloro-pyridin-3-ylamino)-pyrimidin-4-yl]-4-méthyl-thiazol-5-yl}-éthanone
[4-(4,5-diméthyl-thiazol-2-yl)-pyrimidin-2-yl]-(6-méthoxy-pyridin-3-yl)-amine
(6-chloro-pyridin-3-yl)-[4-(4,5-diméthyl-thiazol-2-yl)-pyrimidin-2-yl]-amine
(6-pyrrolidin-1-yl-pyridin-3-yl)-(4-thiazol-2-yl-pyrimidin-2-yl)-amine
2-[2-(6-chloro-pyridin-3-ylamino)-pyrimidin-4-yl]-4-hydroxy-thiazole-5-carboxylate d'éthyle
2-[2-(6-chloro-pyridin-3-ylamino)-pyrimidin-4-yl]-5-méthyl-thiazol-4-ol
2-[2-(6-chloro-pyridin-3-ylamino)-pyrimidin-4-yl]-5-(2-hydroxy-éthyl)-thiazol-4-ol
(6-chloro-5-méthyl-pyridin-3-yl)-(4-thiazol-2-yl-pyrimidin-2-yl)-amine.

8. Composé selon la revendication 1 qui est choisi parmi les suivants :
(6-méthoxy-pyridin-3-yl)-(4-thiazol-2-yl-pyrimidin-2-yl)-amine
(6-chloro-pyridin-3-yl)-(4-thiazol-2-yl-pyrimidin-2-yl)-amine
[4-(4,5-diméthyl-thiazol-2-yl)-pyrimidin-2-yl]-(6-méthoxy-pyridin-3-yl)-amine
(6-pyrrolidin-1-yl-pyridin-3-yl)-(4-thiazol-2-yl-pyrimidin-2-yl)-amine
2-[2-(6-chloro-pyridin-3-ylamino)-pyrimidin-4-yl]-4-hydroxy-thiazole-5-carboxylate d'éthyle
2-[2-(6-chloro-pyridin-3-ylamino)-pyrimidin-4-yl]-5-méthyl-thiazol-4-ol
2-[2-(6-chloro-pyridin-3-ylamino)-pyrimidin-4-yl]-5-(2-hydroxy-éthyl)-thiazol-4-ol
(6-chloro-5-méthyl-pyridin-3-yl)-(4-thiazol-2-yl-pyrimidin-2-yl)-amine.

9. Composé selon la revendication 1 qui est choisi parmi les suivants :
(6-méthoxy-pyridin-3-yl)-(4-thiazol-2-yl-pyrimidin-2-yl)-amine ; et
(6-chloro-pyridin-3-yl)-(4-thiazol-2-yl-pyrimidin-2-yl)-amine
(6-pyrrolidin-1-yl-pyridin-3-yl)-(4-thiazol-2-yl-pyrimidin-2-yl)-amine
2-[2-(6-chloro-pyridin-3-ylamino)-pyrimidin-4-yl]-4-hydroxy-thiazole-5-carboxylate d'éthyle
2-[2-(6-chloro-pyridin-3-ylamino)-pyrimidin-4-yl]-5-méthyl-thiazol-4-ol
2-[2-(6-chloro-pyridin-3-ylamino)-pyrimidin-4-yl]-5-(2-hydroxy-éthyl)-thiazol-4-ol
(6-chloro-5-méthyl-pyridin-3-yl)-(4-thiazol-2-yl-pyrimidin-2-yl)-amine.

10. Composé selon la revendication 1 qui est la (6-chloro-pyridin-3-yl)-(4-thiazol-2-yl-pyrimidin-2-yl)-amine.

11. Composé de formule Ia, ou sel pharmaceutiquement acceptable de celui-ci, 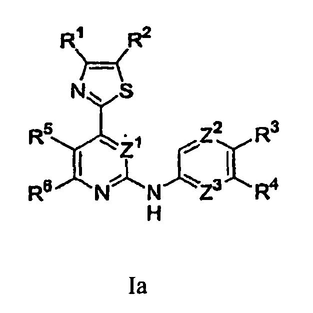 où :
X¹ est N;
R¹ est alkyle, aryle, OH ou (CH₂)ₐCOOR¹⁵;
R² est COR¹⁸, H, COOR¹⁵ ou alkyle ;
R³ est halo, H, OH, alkyle ou morpholino ;
R⁴ est H, NH₂, OH, CF₃ ou NO₂; et
Z² et Z³ sont l'un et l'autre CH ;
a est 0, 1,2,3 ou 4 ;
R¹⁵ et R¹⁸ sont chacun indépendamment H ou C₁₋₆-alkyle.

12. Composé selon la revendication 11 où :
R¹ est Me, Ph, OH ou CH₂COOMe ;
R² est COMe, H, COOEt ou Me ; et
R³ est halo, H, OH, C₁₋₆-alkyle ou morpholino.

13. Composé selon la revendication 11 qui est choisi parmi les suivants :
1-{2-[2-(4-chloro-phénylamino)-pyrimidin-4-yl]-4-méthyl-thiazol-5-yl}-éthanone
(4-chloro-phényl)-[4-(4-méthyl-thiazol-2-yl)-pyrimidin-2-yl]-amine
(4-chloro-phényl)-[4-(4-phényl-thiazol-2-yl)-pyrimidin-2-yl]-amine
2-[2-(4-chloro-phénylamino)-pyrimidin-4-yl]-4-méthyl-thiazole-5-carboxylate d'éthyle
{2-[2-(4-chloro-phénylamino)-pyrimidin-4-yl]-thiazol-4-yl} -acétate de méthyle
2-[2-(4-chloro-phénylamino)-pyrimidin-4-yl]-4-hydroxy-thiazole-5-carboxylate d'éthyle
N-[4-(4,5-diméthyl-thiazol-2-yl)-pyrimidin-2-yl]-benzène-1,3-diamine
3-[4-(4,5-diméthyl-thiazol-2-yl)-pyrimidin-2-ylamino]-phénol
[4-(4,5-diméthyl-thiazol-2-yl)-pyrimidin-2-yl]-(3-trifluorométhyl-phényl)-amine
(4-chloro-3-trifluorométhyl-phényl)-[4-(4,5-diméthyl-thiazol-2-yl)-pyrimidin-2-yl]-amine
[4-(4,5-diméthyl-thiazol-2-yl)-pyrimidin-2-yl]-(4-nitro-phényl)-amine
[4-(4,5-diméthyl-thiazol-2-yl)-pyrimidin-2-yl]-(4-morpholin-4-yl-phényl)-amine
[4-(4,5-diméthyl-thiazol-2-yl)-pyrimidin-2-yl]-(4-méthyl-3-nitro-phényl)-amine
4-[4-(4,5-diméthyl-thiazol-2-yl)-pyrimidin-2-ylamino]-phénol.

14. Composition pharmaceutique comprenant un composé selon l'une quelconque des revendications précédentes avec un diluant, excipient ou vecteur pharmaceutiquement acceptable.

15. Utilisation d'un composé selon l'une quelconque des revendications 1 à 13 dans la préparation d'un médicament pour traiter une affection proliférative.

16. Utilisation selon la revendication 15 où l'affection proliférative est un cancer ou une leucémie.

17. Utilisation selon la revendication 15 où l'affection proliférative est la glomérulonéphrite, la polyarthrite rhumatoïde, le psoriasis ou une affection pulmonaire obstructive chronique.

18. Utilisation d'un composé selon l'une quelconque des revendications 1 à 13 dans la préparation d'un médicament pour traiter une affection virale.

19. Utilisation selon la revendication 18 où l'affection virale est choisie parmi le cytomégalovirus humain (HCMV), l'herpèsvirus de type 1 (HSV-1), le virus d'immunodéficience humaine de type 1 (HIV-1) et le virus varicelle-zona (VZV).

20. Utilisation d'un composé selon l'une quelconque des revendications 1 à 13 dans la préparation d'un médicament pour traiter une affection du SNC.

21. Utilisation selon la revendication 20 où l'affection du SNC est la maladie d'Alzheimer ou une affection bipolaire.

22. Utilisation d'un composé selon l'une quelconque des revendications 1 à 13 dans la préparation d'un médicament pour traiter l'alopécie.

23. Utilisation d'un composé selon l'une quelconque des revendications 1 à 13 dans la préparation d'un médicament pour traiter une attaque.

24. Utilisation d'un composé selon l'une quelconque des revendications 1 à 13 dans la préparation d'un médicament pour traiter le diabète.

25. Utilisation selon la revendication 24 où le diabète est le diabète non insulino-dépendant ou le diabète de type II.

26. Utilisation d'un composé selon l'une quelconque des revendications 1 à 13 dans la préparation d'un médicament pour traiter une maladie inflammatoire ou une maladie infectieuse.

27. Utilisation d'un composé selon l'une quelconque des revendications 1 à 13 dans une analyse pour identifier d'autres composés candidats capables d'inhiber une ou plusieurs d'une kinase dépendante de cycline, d'une kinase aurora, de GSK et d'une enzyme PLK.

28. Utilisation selon la revendication 27 où ladite analyse est une analyse à liaison compétitive.

29. Procédé pour préparer un composé de formule I selon la revendication 1 ou un composé de formule Ia selon la revendication 11, ledit procédé comprenant la réaction d'un composé de formule 9 avec un composé de formule 10 pour former un composé de formule I, où les R¹⁻⁶ sont définis comme précédemment

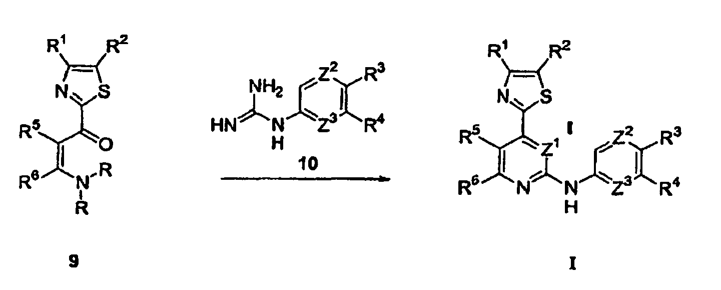

30. Procédé pour préparer un composé de formule I selon la revendication 1 ou un composé de formule Ia selon la revendication 11, ledit procédé comprenant la réaction d'un composé de formule 15 avec un composé de formule 3 pour former un composé de formule I, où les R¹⁻⁶ sont définis comme précédemment
